# EUROPEAN PATENT APPLICATION

(11) **EP 4 799 603 A1**
(43) Date of publication of application: **02.09.2026**
(21) Application number: 24882314.8
(22) Date of filing: 18.10.2024
(51) Int. Cl.: A61K 8/29, A61K 8/34, A61K 8/894, A61Q 1/04, A61Q 1/06, A61Q 1/10, A61Q 1/12, A61Q 17/04, A61Q 19/00

(54) **DISPERSION AND COSMETIC CONTAINING SAME**

(30) Priority: 26.10.2023 JP 2023183903
(71) Applicant: SHIN-ETSU CHEMICAL CO., LTD., Tokyo 100-0005 (JP)
(72) Inventor: MIYAUCHI Masaru, Tokyo 100-0005 (JP); KONISHI Masayuki, Tokyo 100-0005 (JP)
(74) Representative: Mewburn Ellis LLP
(86) International application number: PCT/JP2024/037215
(87) International publication number: WO 2025/089201

(57) **Abstract**

Provided is a dispersion having excellent dispersibility, especially dispersibility in aqueous media, water resistance, and feel on use (absence of stickiness) that contains components (a)-(d):
(a) 10-85 mass% of a hydrophobized inorganic powder having a number-average primary particle size by image analysis of a transmission electron micrograph of more than 200 nm and equal to or less than 700 nm,
(b) 1-80 mass% of an aqueous component having two or more alcoholic hydroxyl groups,
(c) 1-20 mass% of a polyglycerol-modified silicone that is soluble in component (b), and
(d) 0-2 mass% of a polyglycerol-modified silicone that is insoluble in component (b), where the ratio of the content of component (d) to the content of component (c) represented by (d)/(c) is 0.1 or less.

## Description

### TECHNICAL FIELD

The present invention relates to a dispersion containing a hydrophobized inorganic powder and to a cosmetic composition containing the dispersion.

### BACKGROUND ART

Inorganic powders, such as titanium dioxide and iron oxide, are generally used in makeup cosmetic compositions. These inorganic powders effectively improve impression by giving high coverage to conceal skin problems, and by making the skin appear brighter and more rosy.

These inorganic powders are used in the form of dispersion in order to be well dispersed in cosmetic compositions and to enhance the feeling on use of cosmetic compositions. Various techniques have been developed for dispersing such hydrophobically treated inorganic powders (Patent Document 1). In particular, polyglycerin-modified silicones are known to allow the powders to exhibit high dispersibility (Patent Document 2). However, no studies have been made on dispersing inorganic powders in aqueous media using polyglycerin-modified silicones.

These inorganic powders are often hydrophobized on the surface so that the powders can be blended into an oil phase of a cosmetic composition or their water resistance will be enhanced. The addition of such surface-hydrophobized powders is expected to provide makeup effects that can be maintained even when wetted with water (Patent Document 3). On the other hand, the inorganic powders are sometimes hydrophilized on the surface with silica or the like when the powders are to be blended into an aqueous phase. Unfortunately, such hydrophilized powders have drawbacks in water resistance and the feeling on use. Studies on dispersing hydrophobized powders in water have led to the use of a polyether-modified silicone in a dispersion of the hydrophobized inorganic powder. However, such dispersions of hydrophobized inorganic powders have drawbacks in water resistance and the feeling on use (Patent Documents 4 and 5).

Furthermore, a technique is known in which a dispersible powder is obtained by surface-treating a powder with a silicone surfactant. However, the dispersibility and the water resistance of inorganic powders have not been studied sufficiently (Patent Document 6). For these reasons, there has been a demand for a dispersion that shows enhanced dispersibility and water resistance and offers a good feeling on use when blended into a cosmetic composition.

### PRIOR ART DOCUMENTS

### PATENT DOCUMENTS

Patent Document 1: JP-A 2016-079148
Patent Document 2: JP-A 2004-169015
Patent Document 3: JP-A 2021-001213
Patent Document 4: JP-A 2017-218433
Patent Document 5: WO 2017/203846
Patent Document 6: JP-A 2020-002031

### SUMMARY OF INVENTION

### TECHNICAL PROBLEM

The present invention has been made in consideration of the circumstances discussed above. It is therefore an object of the present invention to provide a dispersion that shows excellent dispersibility, particularly in an aqueous medium, and exhibits excellent water resistance and comfortable feeling on use (non-stickiness). Another object of the present invention is to provide a cosmetic composition that includes the dispersion and has high stability, excellent water resistance, and comfortable feeling on use (non-stickiness).

### SOLUTION TO PROBLEM

The present inventors carried out extensive studies directed to solving the problems described above. As a result, the present inventors have found that a dispersion that shows excellent dispersibility, particularly in an aqueous medium, and exhibits excellent water resistance and comfortable feeling on use (non-stickiness) can be obtained by formulating the dispersion using components including the following components (a) to (d):
(a) 10 to 85% by mass of a hydrophobized inorganic powder having a number average primary particle diameter of more than 200 nm and 700 nm or less as determined by image analysis of a transmission electron photomicrograph;
(b) 1.0 to 80% by mass of an aqueous component having at least two alcoholic hydroxyl groups;
(c) 1.0 to 20% by mass of a polyglycerin-modified silicone soluble in the component (b); and
(d) 0 to 2% by mass of a polyglycerin-modified silicone insoluble in the component (b),
in such a manner that the ratio of the content of the component (d) to the content of the component (c), namely, (d)/(c) is 0.1 or less. The present inventors have further found that this dispersion can be blended into a cosmetic composition without causing problems, such as viscosity drop or gelation, and the cosmetic composition that is obtained is highly stable and exhibits excellent water resistance and comfortable feeling on use (non-stickiness). The present invention has been completed based on the findings.

Accordingly, the present invention provides a dispersion and a cosmetic composition containing the dispersion, as defined below:
1. A dispersion including the following components (a) to (d):
   (a) 10 to 85% by mass of a hydrophobized inorganic powder having a number average primary particle diameter of more than 200 nm and 700 nm or less as determined by image analysis of a transmission electron photomicrograph;
   (b) 1 to 80% by mass of an aqueous component having at least two alcoholic hydroxyl groups;
   (c) 1 to 20% by mass of a polyglycerin-modified silicone soluble in the component (b); and
   (d) 0 to 2% by mass of a polyglycerin-modified silicone insoluble in the component (b),
   the ratio of the content of the component (d) to the content of the component (c), (d)/(c), being 0.1 or less.
2. The dispersion according to 1, wherein the component (a) is an inorganic powder hydrophobized with a silicone.
3. The dispersion according to 2, wherein the silicone is triethoxysilylethyl polydimethylsiloxyethylhexyl dimethicone.
4. The dispersion according to any one of 1 to 3, wherein the content of the component (a) is 70 to 85% by mass.
5. The dispersion according to any one of 1 to 4, wherein the component (a) is a coloring pigment.
6. The dispersion according to any one of 1 to 5, wherein the component (c) is polyglyceryl-3 disiloxane dimethicone.
7. The dispersion according to any one of 1 to 6, wherein the content of the component (c) is 3.0 to 8.0% by mass.
8. The dispersion according to any one of 1 to 7, wherein the ratio of the content of the component (c) to the content of the component (b), (c)/(b), is 0.1 to 0.4.
9. A cosmetic composition including the dispersion described in any one of 1 to 8.

### ADVANTAGEOUS EFFECTS OF INVENTION

According to the present invention, a dispersion of an inorganic powder can be provided that shows excellent dispersibility, particularly in an aqueous medium, and exhibits excellent water resistance and comfortable feeling on use (non-stickiness). In the dispersion provided herein, in particular, even titanium oxide fine particles that are difficult to disperse can be dispersed with high stability. The present invention can further provide a cosmetic composition that includes the dispersion and has high stability, excellent water resistance, and comfortable feeling on use (non-stickiness).

### DESCRIPTION OF EMBODIMENTS

Now the present invention is described in detail. Herein, the name of ingredients is sometimes expressed by the Japanese labeling name or the International Nomenclature of Cosmetic Ingredients (INCI). When the Japanese labeling name is identical with the INCI, sometimes either one is omitted.

### [Components (a)]

The component (a) in the present invention is a hydrophobized inorganic powder having a number average primary particle diameter of more than 200 nm and 700 nm or less as determined by image analysis of a transmission electron photomicrograph. Such powders may be used singly, or two or more may be used in combination.

The number average primary particle diameter of the component (a) as determined by image analysis of a transmission electron photomicrograph is more than 200 nm and 700 nm or less, and is preferably 220 to 650 nm, and more preferably 240 to 600 nm. If the particle diameter is larger than 700 nm, the component may give a heavy feeling on use. If the particle diameter is 200 nm or less, the component may give rise to a dry or squeaky feel to deteriorate the feeling on use. The average primary particle diameter of the component (a) in the present invention is the number average diameter measured by image analysis based on a transmission electron photomicrograph. When the powder is not spherical, the number average primary particle diameter is the average of the minor axe of the particles.

The inorganic powder may be any ingredient that can be commonly added to cosmetic compositions. The inorganic powders may be used singly, or two or more may be used in combination. Specific examples include coloring pigments. The coloring pigments are not particularly limited as long as they are commonly used in cosmetics for the purpose of coloring. Examples include red iron oxide (labeling name, INCI: Iron Oxides), yellow iron oxide (labeling name, INCI: Iron Oxides), white titanium dioxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Diooxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium dioxide (labeling name, INCI: Titanium/Titanium Dioxide), Li/cobalt titanate (labeling name, INCI: Lithium Cobalt Titanate), sintered iron oxide/titanium dioxide (labeling name), composites doped with a different metal, such as iron oxide-doped titanium dioxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as γ-iron oxide, inorganic yellow pigments, such as loess, and colored pigments, such as lake form of tar dyes and lake form of natural dyes. Any of the foregoing may be used. The pigment may take any shape including spherical, generally spherical, bar, spindle, petal, strip, and irregular shapes. Its geometry is not particularly limited as long as a color can be imparted to a cosmetic composition.

In order to weaken aggregation or to reduce the activity of the powder, the inorganic powder may be surface-treated with, for example, silica, hydrous silica, alumina, or aluminum hydroxide, before the hydrophobic treatment. The use of alumina or aluminum hydroxide as the agent for the above surface treatment is not preferable in the case where there is a concern that the surface treatment agent may inhibit the swelling of a water-soluble polymer compound added together with the powder in a cosmetic composition, or may cause a loss of water resistance. It is preferable that the above treatment before the hydrophobic treatment be not performed, or the powder be a composite powder that has been surface-treated with hydrous silica that suppresses the activity of the powder and is unlikely to inhibit the swelling of a water-soluble polymer compound.

The hydrophobized inorganic powder is obtained by hydrophobic treatment. The hydrophobic treatment agent is not particularly limited and may be any known treatment agent generally used in cosmetics. Examples include silanes or silylating agents, such as triethoxycaprylylsilane (AES-3083, manufactured by Shin-Etsu Chemical Co., Ltd.) and aminopropyltriethoxysilane, silicones, specifically, dimethyl silicone (KF-96AK series, manufactured by Shin-Etsu Chemical Co., Ltd.), methylhydrogen polysiloxanes, such as hydrogen dimethicone (for example, KF-99P and KF-9901, manufactured by Shin-Etsu Chemical Co., Ltd.), and silicone-branched silicones, such as triethoxysilylethyl polydimethylsiloxyethyl dimethicone and triethoxysilylethyl polydimethylsiloxyethylhexyl dimethicone (for example, KF-9908 and KF-9909, manufactured by Shin-Etsu Chemical Co., Ltd.), waxes, paraffins, organic fluorine compounds, such as perfluoroalkyl phosphate salts, surfactants, N-acylglutamic acids and salts thereof, amino acids, such as lauroyl lysine and sodium lauroyl aspartate, esters, such as isostearyl sebacate and polyglyceryl triisostearate, metal soaps, such as aluminum stearate and magnesium myristate, and isopropyl titanium triisostearate. In particular, silicone treatment is preferrable in terms of the dispersibility with the component (c). More preferably, the component includes one or more selected from triethoxycaprylylsilane, triethoxysilylethyl polydimethylsiloxyethyl dimethicone, triethoxysilylethyl polydimethylsiloxyethylhexyl dimethicone, and hydrogen dimethicone. Triethoxysilylethyl polydimethylsiloxyethylhexyl dimethicone is most preferred. The hydrophobic treatment method is not particularly limited and may be any well-known method, with examples including wet treatment methods, dry treatment methods, and gas phase methods.

Specific examples of the hydrophobized coloring pigments include KTP-09 series, especially KTP-09W, KTP-09R, KTP-09Y, and KTP-09B, from Shin-Etsu Chemical Co., Ltd.

The content of the component (a) is 10 to 85% by mass, preferably 40 to 85% by mass, more preferably 70 to 85% by mass, and still more preferably 75 to 80% by mass of the dispersion. If the content is less than 10% by mass, the dispersion may decrease its viscosity and the age stability of the dispersion may be deteriorated. If the component (a) is blended in an amount larger than 85% by mass, there is a risk that spreadability on use is aggravated or the dispersion loses age stability and experiences a viscosity buildup.

### [Components (b)]

The component (b) in the present invention is an aqueous component having at least two alcoholic hydroxyl groups, specifically, a component that dissolves in water at 25°C. Such components may be used singly, or two or more may be used in combination. The component (b), when blended, helps the component (c) uniformly orient on the surface of the component (a). Specific examples include sugar alcohols, such as Sorbitol (INCI), Maltose (INCI), Xylitol (INCI), Glucose (INCI), Glyceryl Glucoside (INCI), sodium chondroitin sulfate (labeling name, INCI: Sodium Chondroitin Sulfate), Methyl Gluceth-10 (INCI), Methyl Gluceth-20 (INCI), hyaluronic acid, phosphatidyl glycerol, and phosphatidyl inositol; glycols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), and 1,2-Hexanediol (INCI); and polyhydric alcohols, such as Erythritol (INCI), Glycerin (INCI), Diglycerin (INCI), and polyethylene glycol. Of these, such glycols as BG (butylene glycol) and DPG (dipropylene glycol), and such polyhydric alcohols as glycerin are preferable because they dissolve in water in any proportion and also in view of the feeling on use and the versatility as cosmetic ingredients. In particular, glycols having two alcoholic hydroxyl groups in the molecule are more preferable.

The content of the component (b) is 1.0 to 80% by mass, preferably 5 to 30% by mass, and more preferably 10 to 25% by mass of the dispersion. From the point of view of water resistance, the content is more preferably 7 to 15% by mass. From the point of view of age stability, the content is more preferably 15 to 25% by mass, and still more preferably 15 to 20% by mass. If the content is less than 1.0% by mass, the dispersion becomes less stable. Controlling the content to 30% by mass or less is advantageous in that the stickiness of a cosmetic composition can be further reduced, and that the control avoids excessive addition of the component (b) and ensures that the component (b) will not inhibit the orientation of the component (c), which will be described later, on the component (a), thus making it possible to avoid an excessively high viscosity and to achieve higher dispersibility in an aqueous medium.

### [Components (c)]

The component (c) in the present invention is a polyglycerin-modified silicone that is soluble in the component (b). Such components may be used singly, or two or more may be used in combination. By the use of the polyglycerin-modified silicone that is soluble in the component (b), the polyglycerin-modified silicone can be blended in water and can function as a dispersant for the hydrophobized inorganic powder in water. Regarding the phrase that the component (c) is "soluble in the component (b)", the component (c) is "soluble" when a mixture of 20% by mass of the component (c) with the component (b), after being allowed to stand at 25°C, is transparent to semi-transparent without any boundary, whereas the component (c) is "insoluble" when the mixture becomes white turbid or separates into two layers. Being "transparent to semi-transparent" means that the total light transmission through a 1 cm thick cell filled with the mixture liquid is 50% or more as measured in accordance with JIS K7361-1: 1997. When the component (b) is solid at 25°C, the component (c) is dissolved at or above the melting point and the solubility is visually judged in the same manner as above.

The chemical structure of the polyglycerin-modified silicone (c) may be such that the silicone chain as the backbone is modified with a block or a graft of polyglycerin. In order to maintain uniform dispersibility of the component (a) in a cosmetic composition, it is more preferable to use a branch-modified silicone. The silicone backbone may have a branched chain, such as a silicone chain. Specific examples include Polyglyceryl-3 Disiloxane Dimethicone (INCI). From the point of view of water resistance, the component (c) is preferably one that dissolves at 25°C when mixed in BG at a concentration of 20% by mass, and does not dissolve at 25°C when mixed in water at a concentration of 20% by mass. The polyglycerin-modified silicone in the present invention may be co-modified with hydrophilic groups other than polyglycerin groups, but 50% by mass or more of the hydrophilic groups are polyglycerin groups. From the point of view of water resistance, silicone exclusively modified with polyglycerin groups is preferable.

The polyglycerin-modified silicone (c) used in the present invention preferably has an HLB (hydrophile-lipophile balance) value of 3 or more and less than 17, more preferably 5 or more and less than 15, and still more preferably 8 or more and less than 12. If the value is less than 3, the affinity for water is so low that dispersibility is lowered. If the value is 17 or more, there is a concern that water resistance may be low. The HLB is calculated from "(sum of formula weights of hydrophilic group moieties/total molecular weight) × 20".

The content of the component (c) is 1.0 to 20% by mass, preferably 1 to 15% by mass, more preferably 2 to 10% by mass, and still more preferably 3 to 8% by mass of the dispersion. If the content is less than 1.0% by mass, the dispersion may become less stable. If the content is more than 20% by mass, molecules of the component (c) may be oriented on the component (a) that already has molecules of the component (c) oriented thereon, or molecules of the component (c) that have been oriented on the component (a) may be easily detached by other molecules of the component (c) not oriented on the component (a). Possible consequences are an increased viscosity of the dispersion and adverse effects on dispersibility in an aqueous medium.

Furthermore, the ratio (c)/(b) of the content of the component (c) to the content of the component (b) is preferably 0.1 to 1.2. From the points of view of dispersibility and water resistance, in particular, the ratio is preferably 0.1 to 0.7, and more preferably 0.1 to 0.4. By controlling the ratio to 0.1 or more, the component (c) can orient on the component (a) while suffering less inhibition by the component (b), and the dispersion is more unlikely to experience a viscosity buildup and becomes more stable. Controlling the ratio to 1.2 or less ensures that the component (c) is dispersed more sufficiently in the dispersion medium and can easily orient on the component (a), and the dispersion is more unlikely to experience a viscosity buildup and becomes more stable.

### [Components (d)]

The component (d) in the present invention is a polyglycerin-modified silicone that is insoluble in the component (b). Such components may be used singly, or two or more may be used in combination. The definition of "insoluble in the component (b)" is as described above. The component (d) is a polyglycerin-modified silicone distinct from the component (c). Examples thereof include non-crosslinked polyglycerin-modified silicones and crosslinked polyglycerin-modified silicones.

The chemical structure of the non-crosslinked polyglycerin-modified silicone as the component (d) may be such that the silicone chain as the backbone is modified with a block or a graft of polyglycerin. The silicone backbone may have a branched chain, such as a silicone chain. Specific examples include Polyglyceryl-3 Polydimethylsiloxyethyl Dimethicone (INCI), Lauryl Polyglyceryl-3 Polydimethylsiloxyethyl Dimethicone (INCI), and Isostearyl Polyglyceryl-3 Dimethicone (INCI). Commercially available examples include KF-6104, KF-6106, KF-6105, KF-6180, and KF-6115 manufactured by Shin-Etsu Chemical Co., Ltd.

Examples of the crosslinked polyglycerin-modified silicones as the components (d) include (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI). Commercially available examples of the crosslinked silicone surfactants that are swollen with a liquid oil include KSG-710, KSG-790, KSG-810, KSG-820, KSG-830, KSG-840, KSG-820Z, and KSG-850Z manufactured by Shin-Etsu Chemical Co., Ltd.

The amount in which the component (d) is added is 0 to 2% by mass, preferably 0 to 1% by mass, of the dispersion. The content of the component (d) may be nil. The ratio (d)/(c) of the content of the component (d) to the content of the component (c) is 0.1 or less, preferably 0.10 or less, and more preferably 0.08 or less. From the point of view of dispersibility, in particular, the ratio is preferably 0.05 or less, and still more preferably 0. If the ratio is more than 0.1, the component (d) interacts with the component (c) to inhibit the component (c) from orienting on the powder, thereby deteriorating the dispersibility in water. Furthermore, the dispersion may experience a viscosity buildup and may become less stable.

In the dispersion of the present invention, the total content of the components (a), (b), (c), and (d) is preferably 80 to 100% by mass, more preferably 90 to 100% by mass, and still more preferably 99 to 100% by mass. By controlling the total content of the components (a), (b), (c), and (d) to 80% by mass or more, the dispersion that is prepared can attain good stability and comfortable feeling on use. If the total content is less than 80% by mass, there is a risk that the stability of the dispersion can be adversely affected by components other than the components (a), (b), (c), and (d).

Water may be blended into the dispersion of the present invention. When water is blended, the amount thereof is preferably 0 to 20% by mass of the dispersion. It is more preferable that water be not blended.

An antifoaming agent may be added to the dispersion to enhance handling properties at the time of preparation, filling, or the like. Examples of the antifoaming agents include Simethicone (INCI), Dimethicone (INCI), salts, such as sodium chloride (labeling name, INCI: Sodium Chloride), and polyether-modified silicones. Oils, such as dimethicone, may be used as previously emulsified emulsion types, and compounds, such as simethicone, may be used as self-emulsion types previously mixed with silicone surfactants or the like. Among those described above, in particular, simethicone and sodium chloride are preferable in terms of defoaming properties and antifoaming properties. Simethicone is most preferable in terms of the affinity for cosmetic ingredients. The amount in which the antifoaming agent is added is preferably 0.0001 to 1% by mass, more preferably 0.0025 to 0.6% by mass, and still more preferably 0.005 to 0.01% by mass of the whole dispersion.

### [Dispersions]

The dispersion of the present invention is easy to blend into an aqueous medium. The dispersion shows temporary sedimentation when tested by the dispersibility test described later in Examples, but becomes uniformly dispersed in an effective manner upon continued stirring.

### [Properties of the dispersions]

The dispersion of the present invention is preferably a paste or a liquid. In the case where the dispersion is a paste, the hardness is preferably 1 to 180, more preferably 1 to 100, and still more preferably 1 to 50. When the dispersion is a liquid, the viscosity is preferably 10 to 30,000 mPa·s, more preferably 10 to 10,000 mPa·s, and still more preferably 10 to 5,000 mPa·s. If the hardness is greater than 180, the dispersion is difficult to handle. If the viscosity is less than 10 mPa·s, the powder is easily sedimented and the dispersion may be less stable. The hardness is a value measured with a rheometer, for example, Rheometer RT-2002D·D (manufactured by RHEOTECH, probe: 3 mm φ, penetration depth: 10 mm, sample stage rising speed: 5 cm/min, temperature: 25°C, range: 200). The viscosity is measured at 25°C using a Brookfield viscometer (TVB-10, manufactured by Toki Sangyo Co., Ltd.) in accordance with the method described in JIS K7117-1: 1999.

The dispersion of the present invention is preferably dispersible in water. In the present invention, the phrase "dispersible in water" means that when 0.5 g of the dispersion is added to a beaker containing 50 mL of water and the mixture is stirred for less than 2 minutes, the dispersion is uniformly dispersed with no sedimentation. The time required for uniform dispersion is preferably less than 1 minute, and more preferably less than 30 seconds.

### [Methods for producing the aqueous dispersion]

The dispersion of the present invention may be prepared using any known method and apparatus without limitation. For example, use may be made of any agitators, mills, mixers, media agitating mills, planetary centrifugal agitators, and dispersing machines, such as Henschel mixers, ball mills, kneaders, planetary mixers, ribbon blenders, dispersing mixers, homo-mixers, jet mills, roll mills, bead mills, and high-pressure dispersers. From the point of view of mixing efficiency, in particular, it is preferable that the components be dispersed using a roll mill.

### [Cosmetic compositions]

The dispersion of the present invention may be used in a variety of applications and is usable as, in particular, an ingredient for all cosmetic compositions that are externally applied to the skin and hair.

When the dispersion of the present invention is blended into a cosmetic composition, the amount thereof is preferably 0.5 to 60% by mass, more preferably 1.0 to 50% by mass, and still more preferably 5.0 to 40% by mass of the whole of the cosmetic composition. If the amount is less than 0.5% by mass, sufficient makeup effects cannot be expected. If the amount is more than 60% by mass, there is a concern that the feeling on use may be deteriorated.

The type of the cosmetic composition containing the dispersion of the present invention may be any of, for example, water-in-oil emulsion cosmetic, oil-in-water emulsion cosmetic, aqueous cosmetic, and multi-phase emulsions, such as W/O/W and O/W/O. Particularly when the dispersion is used in an oil-in-water emulsion cosmetic, the dispersion is easy to admit and the cosmetic composition that is obtained is stable and has excellent stability, water resistance, and feeling on use.

The form of the cosmetic composition of the present invention may be selected from among liquid, milky lotion, cream, solid, paste, gel, powder, pressed, multilayer, mousse, spray, stick, pencil, and other forms. The multilayer form refers to a cosmetic composition that separates into two or more layers on static holding. Such a cosmetic composition is placed in a container containing stainless steel balls or the like, and is used after shaking the container. Because the dispersion of the present invention has good dispersibility, the cosmetic composition of such form can be readily re-dispersed. The cosmetic composition of such form is called shaking type or the like. Such a cosmetic composition gives a pleasant feeling on use because of easy stabilization, despite the need for shaking. Because the cosmetic composition of the present invention has satisfactory stability, it can also be used without separation into multiple layers. The spray form refers to a spraying cosmetic composition that is placed in a dispenser container, an aerosol container, or the like and is used by being sprayed through a nozzle. The cosmetic composition placed in a dispenser container is sprayed as mist through a nozzle on the dispenser. The cosmetic composition is placed in the aerosol container together with a spraying agent. The spraying agent is not particularly limited and may be selected from, for example, various liquefied petroleum gases (LPG), dimethyl ether, nitrogen gas, and carbon dioxide gas. The spraying agents may be used singly, or two or more may be used in appropriate combination. By virtue of its high dispersibility, the dispersion of the present invention can be used even in this form of cosmetic composition.

The dispersion of the present invention is applicable to a variety of cosmetic compositions, particularly preferably to cosmetic compositions externally applied to the skin, such as skin care cosmetic compositions, make-up cosmetic compositions, antiperspirant cosmetic compositions, and UV-protecting cosmetic compositions, and cosmetic compositions externally applied to the hair, such as hair care cosmetic compositions. Exemplary skin care cosmetic compositions include toilet water, milky lotion, cream, cleansing agent, pack, oil liquid, massage cream, cosmetic liquid, cosmetic oil, detergent, deodorant, hand cream, lip cream, and anti-wrinkle agent. Exemplary make-up cosmetic compositions include make-up foundation, concealer, cosmetic powder, powder foundation, eye color, eye shadow, mascara, eye liner, eye brow cosmetic, and lip cosmetic. Exemplary antiperspirant cosmetic compositions include antiperspirant cosmetic compositions of roll-on, cream, solution, and stick types. Exemplary UV-protecting cosmetic compositions include sunscreen oil, sunscreen milky lotion, and sunscreen cream. Exemplary hair care cosmetic compositions include shampoo, conditioner, treatment, and setting agent. Among these, the make-up cosmetic compositions are particularly preferable.

The cosmetic composition of the present invention may contain various components that are commonly used in conventional cosmetics as long as the advantageous effects of the present invention are not impaired. Suitable components include, for example, (1) oils, (2) aqueous components other than the components (b), (3) surfactants other than the components (c) and (d), (4) powders other than the components (a), (5) compositions consisting of a crosslinked organopolysiloxane and an oil that is liquid at room temperature, (6) film-forming agents, and (7) other additives. These may be used singly, or two or more may be used in appropriate combination. The components described in the dispersion above may be added. The components contained in the dispersion described hereinabove may be added. However, the marked effects aimed at in the present invention cannot be obtained even if the components (a) to (c) of the present invention are added to the cosmetic composition unless the dispersion of the present invention is added.

### (1) Oils

The oils may be volatile or nonvolatile and may be solid, semisolid, or liquid at room temperature (25°C). Examples include silicone oils, silicone waxes, naturally occurring animal and plant oils and fats and semi-synthetic oils and fats, hydrocarbon oils, higher alcohols, fatty acids, ester oils, fluorinated oils, and UV absorbers.

### • Silicone oils

Examples of the silicone oils include Dimethicone (INCI), Trisiloxane (INCI), alkyl-modified silicones, such as Methyl Trimethicone (INCI), Ethyl Trisiloxane (INCI), Ethyl Methicone (INCI), and Hexyl Dimethicone (INCI), long chain alkyl-modified silicones, such as Caprylyl Methicone (INCI), low to high viscosity linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and methyl hydrogen polysiloxane, cyclic organopolysiloxanes, such as Cyclotetrasiloxane (INCI), Cyclopentasiloxane (INCI), and Cyclohexasiloxane (INCI), amino-modified organopolysiloxanes, such as Amodimethicone (INCI) and Aminopropyl Dimethicone (INCI), pyrrolidone-modified organopolysiloxanes, such as PCA Dimethicone (INCI), antifoaming agents, such as Simethicone (INCI), pyrrolidone carboxylic acid-modified organopolysiloxanes, silicone rubbers, such as gum-like dimethylpolysiloxane having a high degree of polymerization, gum-like amino-modified organopolysiloxane, and gum-like dimethylsiloxane-methylphenylsiloxane copolymers, as well as low viscosity organopolysiloxane solutions of silicone gums and rubbers, amino acid-modified silicones, fluorine-modified silicones, silicone resins, and silicone resin solutions.

Commercially available examples of the silicone oils include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

### • Solid oily components

When it is desired that the cosmetic composition of the present invention be solidified, an oily component that is solid at 25°C is preferably blended. Examples of the oily components that are solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), insect wax, shellac wax, and sperm wax; semi-synthetic waxes, such as lanolin ester, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; dioctyldodecyl lauroylglutamate; fatty acids, such as stearic acid and behenic acid; silicone waxes, such as acrylate silicone resins in the form of acrylate silicone graft or block copolymers (such as acrylate silicone graft copolymers: KP-561P and 562P, manufactured by Shin-Etsu Chemical Co., Ltd.); and derivatives thereof. Any one, or two or more compounds selected from the foregoing are preferred.

### • Natural animal and plant oils and semi-synthetic oils

Examples of the natural animal and plant oils and the semi-synthetic oils include naturally occurring plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (California Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, such as corn germ oil (labeling name, INCI: Zea Mays (Corn) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name, INCI: Lanolin Oil).

### • Hydrocarbon oils

Examples of the hydrocarbon oils include linear or branched hydrocarbon oils. The hydrocarbon oils may be volatile or nonvolatile. Specific examples include Olefin Oligomers (INCI), isoparaffins, such as (C13,C14) Isoparaffin (INCI), Isododecane (INCI), Undecane (INCI), Tridecane (INCI), Dodecane (INCI), Isohexadecane (INCI), hydrogenated polyisobutene (labeling name, INCI: Hydrogenated Polyisobutene), Squalane (INCI), Mineral Oil (INCI), and alkanes, such as Coconut Alkanes (INCI) and (C13-15) Alkane (INCI).

### • Higher alcohols

The higher alcohols include, for example, alcohols preferably having 6 or more carbon atoms, more preferably 10 to 30 carbon atoms. Specific examples of the higher alcohols include Lauryl Alcohol (INCI), Myristyl Alcohol (INCI), Palmityl Alcohol (INCI), Stearyl Alcohol (INCI), Behenyl Alcohol (INCI), Oleyl Alcohol (INCI), Isostearyl Alcohol (INCI), Octyl Dodecanol (INCI), Cholesterol (INCI), Phytosterols (INCI), and Batyl Alcohol (INCI).

### • Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and cocoalkyl (caprylate/caprate) (labeling name, INCI: Coco-Caprylate·Caprate).

Glyceride oils are also included in the ester oils, such as triethylhexanoin (labeling name, INCI:), glyceryl tri(caprylate/caprate) (labeling name, INCI: Caprylic/Capric Triglyceride), Cocoglyceryl (INCI), triglyceryl (caprylate/caprate/succinate) (labeling name, INCI: Caprylic/Capric/Succinic Triglyceride), and (caprylic/capric) glycerides (labeling name, INCI: Caprylic/Capric Glycerides).

### • Fluorinated oils

Examples of the fluorinated oils include Perfluorodecalin (INCI), Perfluorononyl Dimethicone (INCI), and Perfluoromethylcyclopentane (INCI).

### • UV absorbers

Suitable UV absorbers include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethyl hexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methylbis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bisethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate).

### (2) Aqueous components other than the components (b)

The aqueous components are not particularly limited as long as they do not belong to the components (b) and are commonly blended in cosmetics. Specific examples include water, lower alcohols preferably having 2 to 5 carbon atoms, such as ethanol (labeling name, INCI: Alcohol) and isopropanol (labeling name, INCI: Isopropyl Alcohol), and humectants, such as Betaine (INCI), PCA-Na (labeling name, INCI: Sodium PCA), egg yolk lecithin, soybean lecithin, phosphatidylcholine, phosphatidylethanolamine, phosphatidylserine, and sphingophospholipid. Also included are water-soluble polymers, for example, gum Arabic, guar gum, carrageenan, agar, quince seed gum, locust bean gum, xanthan gum, pullulan, sodium carboxymethyl cellulose, hydroxyethyl cellulose, vinyl polymers, such as carboxyvinyl polymer, and acrylic polymers, such as (ammonium acryloyldimethyltaurate/vinylpyrrolidone) copolymer, (sodium acrylate/sodium acryloyldimethyltaurate) copolymer, (hydroxyethyl acrylate/sodium acryloyldimethyltaurate) copolymer, (acrylamide/sodium acryloyldimethyltaurate) copolymer, and polyacrylamide. Using acrylic polymers, an oil-in-water cosmetic composition can be stabilized in a relatively easy manner.

### (3) Surfactants other than the components (c) and (d)

The surfactants may be nonionic, anionic, cationic, or ampholytic. The surfactants are not particularly limited as long as they do not belong to the components (c) or (d) and are commonly blended in cosmetics. Of the surfactants, one, or two or more surfactants selected from non-crosslinked silicone surfactants and crosslinked silicone surfactants are preferred because a stable cosmetic composition is obtainable. In any cases, the amount of the surfactant is preferably 0.1 to 20% by mass of the overall cosmetic composition. An amount of at least 0.1% is preferred in that the dispersing and emulsifying functions are fully achieved whereas an amount of up to 20% by mass is preferred because the risk that the cosmetic composition gives a sticky feeling on use is eliminated. The surfactant preferably has an HLB of 2 to 14.5, though not limitative, for the purpose of maintaining the cosmetic composition water resistant.

The non-crosslinked silicone surfactants are linear or branched silicones in which some methyl groups on the backbone are substituted with hydrophilic groups, such as polyethylene glycol. Specifically, preferred such surfactants are linear or branched polyoxyethylene-modified organopolysiloxanes, linear or branched polyoxyethylene polyoxypropylene-modified organopolysiloxanes, linear or branched polyoxyethylene·alkyl-co-modified organopolysiloxanes, linear or branched polyoxyethylene polyoxypropylene·alkyl-co-modified organopolysiloxanes, and linear or branched pyrrolidone-modified organopolysiloxanes. Included are PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), and Cetyl PEG/PPG-10/1 Dimethicone (INCI).

Commercially available examples include KF-6011, KF-6011P, KF-6012, KF-6015, KF-6017, KF-6043, KF-6028, KF-6038, and KF-6048 manufactured by Shin-Etsu Chemical Co., Ltd.

Examples of the crosslinked silicone surfactants include (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI).

On use of the crosslinked silicone surfactant, it is preferred that in a composition consisting of the crosslinked silicone surfactant and an oil that is liquid at room temperature, the crosslinked silicone surfactant swell with the liquid oil as a result of being impregnated with an amount of more than its own weight of the liquid oil.

Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Caprylyl Methicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available examples of the crosslinked silicone surfactants that are swollen with the liquid oil include KSG-210, KSG-240, KSG-270, KSG-310, KSG-320, KSG-330, KSG-340, KSG-320Z, and KSG-350Z from Shin-Etsu Chemical Co., Ltd.

### (4) Powders other than the components (a)

Suitable powders other than the components (a) include fine particle of metal oxide powders, extender pigments, pearly pigments, metal powders, organic powders, and inorganic/organic composite powders, which are exemplified below.

### • Fine particle of inorganic powders

The fine particle of inorganic powders may be any powders that have UV shielding effects and are used as UV scattering agents. Any such ingredients that can be normally blended into cosmetic compositions may be used. Such fine particle of inorganic powders may be used singly, or two or more may be used in combination. In particular, one, or two or more metal oxides selected from titanium dioxide, zinc oxide, and cerium oxide are preferable. The metal oxide may be a composite powder of two or more of titanium dioxide, zinc oxide, and cerium oxide, or may be a composite powder with other powder.

The number average primary particle diameter of the fine particle of inorganic powder determined by image analysis of a transmission electron photomicrograph is 10 to 200 nm, and preferably 150 nm or less. If the particle diameter is more than 200 nm, the UV protection function is lowered and the powder applied leaves a white cast on the skin. If the particle diameter is less than 10 nm, dryness is increased and may adversely affect the feeling on use. The number average primary particle diameter of the fine particle of inorganic powder may be determined based on a transmission electron photomicrograph. When the powder is not spherical, the average primary particle diameter is the average of the minor axe of the particles. The fine particle of inorganic powder may be of spindle, needle, bunch, strip, substantial sphere, or rod shape.

In order to weaken aggregation or to reduce the activity of the powder, the fine particle of inorganic powder may be surface-treated with, for example, silica, hydrous silica, alumina, or aluminum hydroxide, before hydrophobic treatment. In the case where there is a concern that the surface treatment may inhibit the swelling of a water-soluble polymer added together with the powder in a cosmetic composition, or may cause a loss of water resistance, it is preferable that the powder be a composite powder that is not surface-treated with alumina or aluminum hydroxide. When a loss of water resistance is a concern, it is preferable that the powder be a composite powder that is not surface-treated with silica or hydrous silica.

The hydrophobic treatment agent for the above fine particle of inorganic powder is not particularly limited as long as it is a known treatment agent generally used in cosmetic compositions. Examples include silanes or silylating agents, such as caprylylsilane (AES-3083, manufactured by Shin-Etsu Chemical Co., Ltd.), silicon oils, such as dimethyl silicone (KF-96AK series, manufactured by Shin-Etsu Chemical Co., Ltd.), methyl hydrogen polysiloxanes (for example, KF-99P and KF-9901, manufactured by Shin-Etsu Chemical Co., Ltd.), and silicone-branched silicone treatment agents (for example, KF-9908 and KF-9909, manufactured by Shin-Etsu Chemical Co., Ltd.), waxes, paraffins, organic fluorine compounds, such as perfluoroalkyl phosphate salts, surfactants, amino acids, such as N-acylglutamic acid, and metal soaps, such as aluminum stearate and magnesium myristate. In particular, the component including at least one selected from stearic acid, isostearic acid, and triethoxycaprylylsilane is preferable because such an agent can effect satisfactory treatment and ensures good dispersibility. The hydrophobic treatment method is not particularly limited and may be any well-known method, with examples including wet treatment methods, dry treatment methods, and gas phase methods.

The surface-treated fine particle of inorganic powders may be purchased from the market. For example, such fine particle of titanium dioxides are commercially available under the trade names of MT-01, 02, 050OTS, 100Z, 100TV, 100SAS, 150EX, 200ST, 500SAM, 505SAS, 700Z, and 700BS (manufactured by TAYCA Co., Ltd.), ST-455, 455WS, 457ECS, 457SA, 495M, and 455FA (manufactured by Titan Kogyo, Ltd.), and STR-100A-LP, 100C-LP, 100W-LP, 100C-LF, and 40-LP (manufactured by Sakai Chemical Industry Co., Ltd.). Such fine particle of zinc oxides are commercially available under the trade names of MZ-150, 200, 300, 306X, 500HP, 505T, 506X, MZY-203S, 210M3S, TMZ-HA1, and MZX-5080TS (manufactured by TAYCA Co., Ltd.), and FZO-50 (manufactured by ISHIHARA SANGYO KAISHA, LTD.).

### • Extender pigments

Examples of the extender pigments include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), Talc (INCI), Mica (INCI), Kaolin (INCI), synthetic fluorophlogopite (labeling name, INCI: Synthetic Fluorphlogopite), synthetic ferrous golden mica (labeling name), black mica (labeling name, INCI: Biotite), potassium silicate (labeling name, INCI: Potassium Silicate), Silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), Hydroxyapatite (INCI), Bentonite (INCI), Montmorillonite (INCI), Hectorite (INCI), Zeolite (INCI), Alumina (INCI), Al hydroxide (labeling name, INCI: Aluminum Hydroxide), boron nitride (labeling name, INCI: Boron Nitride), and glass (labeling name, INCI: Glass).

### • Coloring pearly pigments

Examples of the inorganic coloring pearly pigments include pearly agents, such as Mica (INCI) coated with titanium dioxide (labeling name, INCI: Titanium Dioxide), and synthetic fluorophlogopite (labeling name, INCI: Synthetic Fluorphlogopite) coated with titanium dioxide (labeling name, INCI: Titanium Dioxide); and pearly pigments, such as bismuth oxychloride (labeling name, INCI: Bismuth Oxychloride), bismuth oxychloride (labeling name, INCI: Bismuth Oxychloride) coated with titanium dioxide (labeling name, INCI: Titanium Dioxide), Talc (INCI) coated with titanium dioxide (labeling name, INCI: Titanium Dioxide), fish scale flakes (labeling name), and coloring mica coated with titanium dioxide (labeling name, INCI: Titanium Dioxide). These may either be untreated or have undergone well-known surface treatment commonly used for cosmetics.

### • Metal powders

Examples of the metal powders include metal microparticles, such as aluminum (labeling name, INCI: Aluminum, Aluminum Powder), copper (labeling name, INCI: Copper Powder), silver (labeling name, INCI: Silver Powder), and gold (labeling name, INCI: Gold).

### • Organic powders

Examples of the organic powders include powders of silicones, polyamides, polyacrylic acid-acrylate, polyesters, Polyethylene (INCI), Polypropylene (INCI), Polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, Cellulose (INCI), Silk (INCI), nylon (labeling name), phenolic resins, epoxy resins, and polycarbonate.

In particular, examples of the silicones include silicone resin particles; Polymethylsilsesquioxane (INCI), silicone rubber powder, silicone resin-coated silicone rubber powder; (Vinyl Dimethicone/Methicone Silsesquioxane) Crosspolymer (INCI), (Diphenyl Dimethicone/Vinyl Diphenyl Dimethicone/Silsesquioxane) Crosspolymer (INCI), Polysilicone-1 Crosspolymer (INCI), and Polysilicone-22 (INCI).

Commercially available examples of the silicone powders include KMP-590, KMP-591, KMP-592, KMP-597, KMP-598, KSP-100, KSP-101, KSP-102, KSP-105, KSP-100W, KSP-300, KSP-411, KSP-441, KM-9729, and KM-440, from Shin-Etsu Chemical Co., Ltd.

Examples further include metal soaps. Specific examples include powders of zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), and K cetylphosphate (labeling name, INCI: Potassium Cetyl Phosphate). Examples further include organic colorants. Specific examples include tar dyes, such as Red #3, Red #104 (1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230 (1) (labeling name, INCI: Red 22, Red 22 Lake), Red #230 (2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202 (1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 (labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), and Orange #207 (labeling name, INCI: Orange 11); and natural dyes, such as Cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powders

Exemplary of the inorganic/organic composite powders is a composite powder obtained by coating an inorganic powder on the surface with an organic powder by any well-known method.

The powders described above may be blended into the dispersion of the present invention as a mixture with the component (a). The amount of the powders other than the component (a) is preferably 20% by mass or less, preferably 10% by mass or less, and more preferably 3% by mass or less of the dispersion of the present invention, and may be 0% by mass. Alternatively, a dispersion of the powders may be prepared separately from the component (a) and blended into the cosmetic composition. This dispersion may or may not contain the components (b), (c), and (d). The amount of the powders other than the component (a) is preferably 20% by mass or less of the cosmetic composition.

### • UV absorbing/scattering agents

A dispersion of UV absorbing/scattering particles previously dispersed in an oil may also be used. Suitable oils include liquid silicone oils, hydrocarbon oils, ester oils, natural animal and plant oils, semi-synthetic oils, and fluorinated oils, all exemplified above as the oils (1) that are optional components.

Specific examples of the dispersions having UV absorbing/scattering particles previously dispersed in an oil include SPD series (product name), especially SPD-T5, SPD-T5L, SPD-Z5, SPD-T6, SPD-Z6, SPD-T7, and SPD-Z7L, from Shin-Etsu Chemical Co., Ltd.

### (5) Compositions consisting of a crosslinked organopolysiloxane and an oil that is liquid at room temperature

In the composition consisting of a crosslinked organopolysiloxane and an oil that is liquid at room temperature, it is preferred that the crosslinked organopolysiloxane swell with the liquid oil as a result of being impregnated with an amount of more than its own weight of the liquid oil. Suitable liquid oils include liquid silicone oils, hydrocarbon oils, ester oils, natural animal and plant oils, semi-synthetic oils, and fluorinated oils, all exemplified above as the oils (1) that are optional components. Illustrative examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), Squalane (INCI), and cocoalkyl (caprylate/caprate) (labeling name, INCI: Coco-Caprylate·Caprate).

Unlike the crosslinked silicone surfactants as the components (3) mentioned above, the components (5) are compounds having neither polyether nor polyglycerin structure in the molecular structure. Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Commercially available examples of the compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, KSG-048Z, and KM-116, from Shin-Etsu Chemical Co., Ltd.

### (6) Film-forming agents

The film-forming agent is blended mainly for the purpose of further maintaining the effect-sustainability of the cosmetic composition. A silicone-based agent is preferred from the aspect of imparting water repellency, though the agent is not limited thereto. Specifically, use may be made of, for example, trimethylsiloxysilicic acid, acrylic-silicone film formers, silicone-modified norbornene, silicone-modified pullulan, and silicone-modified polyvinyl alcohol.

Examples of the film-forming agents in the form of silicone-based agent include trimethylsiloxysilicic acid (labeling name, INCI: Trimethylsiloxysilicate), Acrylates/Dimethicone Copolymer (INCI), Norbornene/Tris-(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan).

The film-forming agent may be previously dissolved in an oil that is liquid at room temperature before it is blended in the cosmetic composition. Suitable liquid oils used herein include liquid silicone oils, hydrocarbon oils, ester oils, natural animal and plant oils, semi-synthetic oils, and fluorinated oils, all exemplified above as the oils (1) that are optional components.

Commercially available examples of the silicone film-forming agents include KF-7312J, KP-545, KP-549, KP-543, NBN-30-ID, TSPL-30-ID, and TSPL-30-D5, from Shin-Etsu Chemical Co., Ltd.

### (7) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

### • Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and α,γ-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals of hectorite, such as Disteardimonium Hectorite (INCI) and Stearalkonium Hectorite (INCI); and Stearalkonium Bentonite (INCI).

### • Preservatives/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, bisabolol, ethylhexylglycerin, glyceryl caprylate, caprylhydroxaminc acid, hexyl dimethylolpropanoate, polyaminopropyl biguanide, photosensitizers, silver, and plant extracts. Preservatives may be used singly, or two or more may be used in combination. In particular, polylysine, bisabolol, ethylhexylglycerin, glyceryl caprylate, caprylhydroxamic acid, hexyl dimethylolpropanoate, polyaminopropyl biguanide, ethylhexylglycerin, and phenoxyethanol are preferable because they are easily blended into the cosmetic composition and are expected to produce preservative effects. Ethylhexylglycerin, phenoxyethanol, and glyceryl caprylate are particularly preferable in terms of the compatibility with the dispersion of the present invention. The addition of preservatives is expected to suppress bacterial contamination and enhance the storage stability of the cosmetic.

### • Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components that exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine).

### • Fragrances

Examples of the fragrances include natural fragrances and synthetic fragrances. Suitable natural fragrances include plant fragrances separated from flowers, leaves, stems, pericarps, and the like; and animal fragrances, such as musk and civet. Suitable synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

### • Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations. In particular, sodium chloride is preferred from the aspects of dissolution, feeling on use, and anti-foaming. When used in large amounts, the salt can inhibit the water-soluble polymer from swelling.

### • Antioxidants

Examples of the antioxidants include, but are not particularly limited to, carotenoid, ascorbic acid and salts thereof, ascorbyl stearate, tocophenol, tocophenol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

### • Acidity regulators

Suitable acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agents

Suitable chelating agents include alanine, sodium EDTA, sodium polyphosphate, sodium metaphosphate, and phosphoric acid.

### • Refreshing agents

Suitable refreshing agents include L-menthol, camphor, and menthyl lactate.

### • Anti-inflammatory agents

Suitable anti-inflammatory agents include allantoin, glycyrrhizinic acid and salts thereof, glycyrrhetinic acid, stearyl glycyrrhetinate, tranexamic acid, and azulene.

### • Skin modifying ingredients

Suitable skin modifying ingredients include brightening or whitening agents, such as placenta extract, arbutin, glutathione, and Saxifraga Sarmentosa Extract; cell activators, such as royal jelly, photosensitizers, cholesterol derivatives, and bovine blood extracts; anti-skin-roughening agents; blood flow enhancing agents, such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

### • Vitamins

Suitable vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as α-tocopherol, β-tocopherol, γ-tocopherol, dl-α-tocopherol acetate, dl-α-tocopherol nicotinate, and dl-α-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

### • Amino acids

Suitable amino acids include glycine, valine, leucine, isoleucine, serine, threonine, phenylalanine, arginine, lysine, aspartic acid, glutamic acid, cystine, cysteine, methionine, and tryptophan.

### • Nucleic acids

Typical of the nucleic acids is deoxyribonucleic acid.

### • Hormones

Suitable hormones include estradiol and ethenyl estradiol.

### • Clathrate compounds

Typical of the clathrate compounds is cyclodextrin.

### EXAMPLES

Examples and Comparative Examples are shown below by way of illustration of the present invention and not by way of limitation. In the Examples below, the compositional percent (%) and the ratio are by mass unless otherwise stated. The amount of product name is the amount of the product blended.

### [Solubility of aqueous dispersants]

Aqueous dispersants were each dissolved in water or BG so that their concentration would be 20%. Their solubilities are described in Table 1 below.

### [Judgement criteria]

A: Soluble
D: Insoluble

The dispersant was judged "Pass" when rated with "A".

### [Solubility of aqueous dispersants]

**[Table 1]**

| | Solubility in water | Solubility in BG |
|---|---|---|
| (c) Polyglyceryl-3 disiloxane dimethicone (HLB 10) | D | A |
| (c) Polyglyceryl-3 disiloxane dimethicone (HLB 12) | A | A |
| (d) Polyglyceryl-3 polydimethylsiloxyethyl dimethicone (HLB 1) | D | D |
| PEG-9 dimethicone | A | A |

### [Examples and Comparative Examples: Dispersions]

Dispersions were obtained using a roll mill based on the formulations described in Tables 2 and 3 below. The dispersibility of the dispersions obtained was evaluated based on the evaluation criteria below. The results are also described in the tables. The dispersions in all of the following examples had a hardness in the range of 1 to 100.

### [Success or failure of the preparation of dispersion]

A: The preparation of dispersion was successful.
D: The preparation of dispersion failed.

The sample was judged "Pass" when rated with "A".

### [Evaluation of dispersibility in water]

The dispersions in the tables below were each collected in an amount of 0.5 g with a metal spatula and added to a beaker containing 50 mL of water. Stirring was then performed to evaluate the dispersibility in water.
A: The dispersion was dispersed uniformly with no sedimentation when stirred for less than 30 seconds.
B: The dispersion was dispersed uniformly when stirred for 30 seconds or more and less than 1 minute.
C: The dispersion was dispersed uniformly when stirred for 1 minute or more and less than 2 minutes.
D: The dispersion was not dispersed uniformly even when stirred for 2 minutes or more. The sample was judged "Pass" when rated with "C" or above.

### [Evaluation of water resistance]

The dispersions obtained above, each in an amount of 0.1 g, were each applied to an area of 5 cm × 5 cm on artificial leather, and dried at 25°C for 24 hours. Thereafter, the water resistance was evaluated according to the following evaluation criteria. The results were judged according to the criterion below.

### [Judgement criteria]

A: Remains after exposed to running water for 1 minute or more.
B: Remains after exposed to running water for 30 seconds or more and less than 1 minute.
C: Vanishes after exposed to running water for 10 seconds or more and less than 30 seconds.
D: Vanishes after exposed to running water for less than 10 seconds.

The sample was judged "Pass" when rated with "C" or above.

### [Evaluation of feeling on use]

The sample was evaluated for the feeling on use (or non-stickiness) on application by a panel of 10 professional members according to the evaluation criteria below. The results of the 10 members were averaged, and the feeling on use was rated according to the judgment criteria below.

### [Evaluation criteria]

5 points: excellent
4 points: good
3 points: moderate
2 points: rather poor
1 point: poor

### [Judgment criteria]

A: The average point is 4.5 points or more.
B: The average point is 3.5 points or more and less than 4.5 points.
C: The average point is 2.5 points or more and less than 3.5 points.
D: The average point is less than 2.5 points.

The sample was judged "Pass" when rated with "C" or above.

**[Table 2]**

| Formulation (%) | Example | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| (a) KTP-09W (Note 1) | 80 | 80 | 80 | 80 | 80 | | | | 72 | 75 | 75 | 70 | 80 |
| (a) KTP-09R (Note 1) | | | | | | 80 | | | 1.5 | | | | |
| (a) KTP-09Y (Note 1) | | | | | | | 80 | | 6 | | | | |
| (a) KTP-09B (Note 1) | | | | | | | | 80 | 0.5 | | | | |
| (b) BG | 18 | 17 | 15 | 12 | 10 | 15 | 15 | 15 | 15 | 23 | 22 | 22 | |
| (b) Glycerin | | | | | | | | | | | | | 15 |
| (c) Polyglyceryl-3 disiloxane dimethicone (HLB10) | 2 | 3 | 5 | 8 | 10 | 5 | 5 | 5 | 5 | 2 | 3 | 8 | 5 |
| PEG-9 dimethicone | | | | | | | | | | | | | |
| (d) Lauryl polyglyceryl-3 polydimethylsiloxyethyl dimethicone | | | | | | | | | | | | | |
| Amodimethicone | | | | | | | | | | | | | |
| Aminopropyl dimethicone | | | | | | | | | | | | | |
| Carboxydecyl dimethicone | | | | | | | | | | | | | |
| Total | 100 | 100 | 100 | 100 | 100 | 100 | 100 | 100 | 100 | 100 | 100 | 100 | 100 |
| (c)/(b) | 0.11 | 0.18 | 0.33 | 0.67 | 1.00 | 0.33 | 0.33 | 0.33 | 0.33 | 0.09 | 0.14 | 0.36 | 0.33 |
| (d)/(c) | 0.00 | 0.00 | 0.00 | 0.00 | 0.00 | 0.00 | 0.00 | 0.00 | 0.00 | 0.00 | 0.00 | 0.00 | 0.00 |
| Success or failure of preparation | A | A | A | A | A | A | A | A | A | A | A | A | A |
| Dispersibility in water | B | A | A | B | C | A | A | A | A | A | A | A | A |
| Water resistance | C | B | A | B | B | A | A | A | A | C | C | C | B |
| Feeling on use | B | A | A | A | C | A | A | A | A | C | A | B | B |

**[Table 3]**

| Formulation (%) | Comparative Example | | | | | | | | | | |
|---|---|---|---|---|---|---|---|---|---|---|---|
| | 1 | 2 | 3 | 4 | 5 | 6 | 7 | 8 | 9 | 10 | 11 |
| (a) KTP-09W (Note 1) | 80 | 90 | 80 | 70 | 70 | 75 | 80 | 80 | 80 | 80 | 80 |
| (a) KTP-09R (Note 1) | | | | | | | | | | | |
| (a) KTP-09Y (Note 1) | | | | | | | | | | | |
| (a) KTP-09B (Note 1) | | | | | | | | | | | |
| (b) BG | 20 | 7 | 19.5 | 5 | 22 | 22 | 15 | 15 | 15 | 15 | 16.5 |
| (b) Glycerin | | | | | | | | | | | |
| (c) Polyglyceryl-3 disiloxane dimethicone (HLB10) | | 3 | 0.5 | 25 | | | | | | | 3 |
| PEG-9 dimethicone | | | | | 8 | 3 | 5 | | | | |
| (d) Lauryl polyglyceryl-3 polydimethylsiloxyethyl dimethicone | | | | | | | | | | | 0.5 |
| Amodimethicone | | | | | | | | 5 | | | |
| Aminopropyl dimethicone | | | | | | | | | 5 | | |
| Carboxydecyl dimethicone | | | | | | | | | | 5 | |
| Total | 100 | 100 | 100 | 100 | 100 | 100 | 100 | 100 | 100 | 100 | 100 |
| (c)/(b) | 0.00 | 0.43 | 0.03 | 5.00 | - | - | - | 0.00 | 0.00 | 0.00 | 0.18 |
| (d)/(c) | - | 0.00 | 0.00 | 0.00 | - | - | - | 0.00 | 0.00 | 0.00 | 0.17 |
| Success or failure of preparation | D | D | A | A | A | A | A | D | D | D | A |
| Dispersibility in water | - | - | D | D | A | A | A | - | - | - | D |
| Water resistance | - | - | A | A | D | D | D | - | - | - | A |
| Feeling on use | - | - | B | D | D | C | C | - | - | - | A |

| | | | | | | | | | | | |
|---|---|---|---|---|---|---|---|---|---|---|---|
| (Note1) Triethoxysilylethyl polydimethylsiloxyethylhexyl dimethicone-treated coloring inorganic pigments, W: white, R: red, Y: yellow, B: black (number average primary particle diameter: 210 to 400 nm), Shin-Etsu Chemical Co., Ltd. (d) Lauryl polyglyceryl-3 polydimethylsiloxyethyl dimethicone: Insoluble in the component (b) | | | | | | | | | | | |

As clear from Table 2, the dispersions (slurries) obtained in Examples of the present invention achieved good dispersibility in water. In contrast, as described in Table 3, the preparation of dispersion failed in Comparative Example 1 that did not involve the component (c) and in Comparative Example 2 in which the amount of the component (a) was excessively large, and Comparative Examples 3 and 4 that involved the component (c) in an amount outside the range of the present invention gave dispersions but were unsatisfactory in dispersibility in water and feeling on use. Comparative Examples 5 to 10 that involved a surfactant not corresponding to the component (c) failed to give a dispersion, or gave a dispersion but was apparently unsatisfactory in water resistance. Furthermore, Comparative Example 11 in which the formulation involved the component (d) but (d)/(c) was more than 0.10 resulted in poor dispersibility in water.

Dispersions of Examples 14 to 18 were obtained using a high-pressure disperser based on the formulations described in Table 4 below. All the dispersions obtained had excellent dispersibility in water, water resistance, and feeling on use.

**[Table 4]**

| Formulation (%) | Example | | | | |
|---|---|---|---|---|---|
| | 14 | 15 | 16 | 17 | 18 |
| (a) KTP-09W (Note 1) | 50 | | | | 50 |
| (a) KTP-09R (Note 1) | | 50 | | | |
| (a) KTP-09Y (Note 1) | | | 50 | | |
| (a) KTP-09B (Note 1) | | | | 50 | |
| Water | 10 | 10 | 10 | | |
| (b) BG | 35 | 35 | 35 | 45 | 44.5 |
| (c) Polyglyceryl-3 disiloxane dimethicone (HLB 10) | 5 | 5 | 5 | 5 | 5 |
| (d) Lauryl polyglyceryl-3 polydimethylsiloxyethyl dimethicone | | | | | 0.5 |
| Total | 100 | 100 | 100 | 100 | 100 |
| (c)/(b) | 0.14 | 0.14 | 0.14 | 0.11 | 0.11 |
| (d)/(c) | 0 | 0 | 0 | 0 | 0.1 |

| | | | | | |
|---|---|---|---|---|---|
| (Note 1) Triethoxysilylethyl polydimethylsiloxyethylhexyl dimethicone-treated coloring inorganic pigments, W: white, R: red, Y: yellow, B: black (number average primary particle diameter: 210 to 400 nm), Shin-Etsu Chemical Co., Ltd. | | | | | |

### [Examples 19 to 34]

Paste-like dispersions were prepared according to the formulations described in Tables 5 and 6.

### (Production method)

Components (1) were dispersed with a roll to give a paste-like dispersion.

**[Table 5]**

| Formulation (%) | | Example | | | | | | | |
|---|---|---|---|---|---|---|---|---|---|
| | | 19 | 20 | 21 | 22 | 23 | 24 | 25 | 26 |
| | (a) KTP-09W (Note 1) | 52.8 | 52.8 | 52.8 | 52.8 | 52.8 | 52.8 | 52.8 | 52.8 |
| (1) | (a) KTP-09Y (Note 1) | 5.9 | 5.9 | 5.9 | 5.9 | 5.9 | 5.9 | 5.9 | 5.9 |
| | (a) KTP-09R (Note 1) | 1.1 | 1.1 | 1.1 | 1.1 | 1.1 | 1.1 | 1.1 | 1.1 |
| | (a) KTP-09B (Note 1) | 0.4 | 0.4 | 0.4 | 0.4 | 0.4 | 0.4 | 0.4 | 0.4 |
| | Silicone-treated talc | 22.0 | | 18.0 | 18.0 | | | | 2.0 |
| | Barium sulfate | | 22.0 | 4.0 | 4.0 | | | | 2.0 |
| | Metal soap-treated fine particle of titanium dioxide (20 nm) | | | | | 10.0 | | 5.0 | 3.0 |
| | Alkylsilane-treated fine particle of zinc oxide (35 nm) | | | | | | 10.0 | 5.0 | 3.0 |
| | (b) BG | 14.8 | 14.8 | 14.8 | 16.8 | 24.8 | 24.8 | 24.8 | 24.8 |
| | (c) KF-6100 (Note 2) | 3 | 3 | 3 | 1 | 5 | 5 | 5 | 5 |
| Total | | 100.0 | 100.0 | 100.0 | 100.0 | 100.0 | 100.0 | 100.0 | 100.0 |

| | | | | | | | | | |
|---|---|---|---|---|---|---|---|---|---|
| (Note 1) KF-9909-treated coloring inorganic pigments, W: white, R: red, Y: yellow, B: black (number average primary particle diameter: 210 to 400 nm), Shin-Etsu Chemical Co., Ltd. (Note 2) Polyglyceryl-3 disiloxane dimethicone, Shin-Etsu Chemical Co., Ltd. | | | | | | | | | |

**[Table 6]**

| Formulation (%) | | Example | | | | | | | |
|---|---|---|---|---|---|---|---|---|---|
| | | 27 | 28 | 29 | 30 | 31 | 32 | 33 | 34 |
| | (a) Hydrogen dimethicone-treated titanium dioxide (Note 1) | 60 | 60 | | | | | | |
| (1) | (a) Triethoxycaprylylsilane-treated titanium dioxide (Note 2) | | | 60 | 60 | | | | |
| | (a) Acrylate silicone-treated titanium oxide (Note 3) | | | | | 60 | 60 | | |
| | (a) Amino acid-treated titanium dioxide (Note 4) | | | | | | | 60 | 60 |
| | Silicone-treated iron oxide (Note 5) | | 5 | | 5 | | 5 | | 5 |
| | (b) BG | 35 | | 35 | | 35 | | 35 | 30 |
| | (b) Pentylene glycol | | 10 | | | | | | |
| | (b) DPG | | | | 30 | | | | |
| | (b) Glycerin | | 20 | | | | 30 | | |
| | (c) KF-6100 (Note 6) | 5 | 5 | 5 | 5 | 5 | 5 | 5 | 5 |
| Total | | 100.0 | 100.0 | 100.0 | 100.0 | 100.0 | 100.0 | 100.0 | 100.0 |

| | | | | | | | | | |
|---|---|---|---|---|---|---|---|---|---|
| (Note 1) Number average primary particle diameter: 250 nm (Note 2) Number average primary particle diameter: 220 nm (Note 3) Number average primary particle diameter: 700 nm (Note 4) Number average primary particle diameter: 500 nm (Note 5) Mixed powder containing KTP-09Y, KTP-09R, and KTP-09B in a ratio of 7:2:1, Shin-Etsu Chemical Co., Ltd. (Note 6) Polyglyceryl-3 disiloxane dimethicone, Shin-Etsu Chemical Co., Ltd. | | | | | | | | | |

### [Example 35] Oil-in-water liquid foundations

| Formulation | | % |
|---|---|---|
| 1. | Glyceryl stearate (SE) | 3.0 |
| 2. | Polysorbate-60 | 3.5 |
| 3. | Sorbitan stearate | 2.0 |
| 4. | KF-7312T (Note 1) | 3.0 |
| 5. | Behenyl alcohol | 2.0 |
| 6. | KF-4418 (Note 2) | 2.5 |
| 7. | Octyldodecyl myristate | 5.0 |
| 8. | Cocoalkyl (caprylate/caprate) | 5.0 |
| 9. | Dimethicone (100 cs) | 0.5 |
| 10. | Ethylhexylglycerin | 0.1 |
| 11. | PCA-Na (50% aqueous solution) | 0.2 |
| 12. | Betaine | 0.1 |
| 13. | Glyceryl glucoside | 0.5 |
| 14. | BG | 8.5 |
| 15. | Water | Balance |
| 16. | KSP-100W (Note 3) | 1.0 |
| 17. | Water | 8.0 |
| 18. | Dispersion of any of Examples 19 to 34 | 6.0 |
| Total | | 100.0 |

| | | |
|---|---|---|
| (Note 1) 60% Solution of trimethylsiloxysilicic acid in methyl trimethicone, Shin-Etsu Chemical Co., Ltd. (Note 2) Caprylyl methicone, Shin-Etsu Chemical Co., Ltd. (Note 3) Hydrophilized (vinyl dimethicone/methicone silsesquioxane) crosspolymer, Shin-Etsu Chemical Co., Ltd. | | |

### (Production method)

A: Components 1 to 9 were mixed and heated to give a uniform solution.
B: Components 10 to 15 were mixed and heated.
C: Components 17 and 18 were mixed.
D: While performing stirring, the mixture obtained in A was added to the mixture obtained in B and emulsified. The mixture obtained in C and component 16 were added. The resultant mixture was mixed to give an oil-in-water liquid foundation.

The oil-in-water liquid foundations obtained as described above showed high stability, a non-sticky pleasant feeling on use, and excellent water resistance. The dispersions of Examples 19 to 34 had excellent stability and were easy to handle and admit.

### [Example 36] Lip cosmetic

| Formulation | | % |
|---|---|---|
| 1. | Candelilla wax | 3 |
| 2. | Polyethylene | 2 |
| 3. | Microcrystalline wax | 2.5 |
| 4. | Ceresin | 6 |
| 5. | KP-561P (Note 1) | 13 |
| 6. | Macadamia nut oil | 23 |
| 7. | Diisostearyl malate | 8.5 |
| 8. | Hydrogenated polyisobutene | 8.5 |
| 9. | Isotridecyl isononanoate | 15 |
| 10. | PEG-75 | 4.5 |
| 11. | KF-6100 (component (c)) | 2.5 |
| 12. | Red #201 | 0.4 |
| 13. | Red #202 | 0.4 |
| 14. | Yellow #4 | 1.6 |
| 15. | KTP-09W (Note 2) | 3 |
| 16. | KTP-09R (Note 2) | 0.7 |
| 17. | KTP-09B (Note 2) | 0.2 |
| 18. | KF-9909-treated (Note 3) talc | 0.7 |
| 19. | Mica | 4.5 |
| Total | | 100.0 |

| | | |
|---|---|---|
| (Note 1) (Acrylates/stearyl acrylate/dimethicone methacrylate) copolymer, Shin-Etsu Chemical Co., Ltd. (Note 2) KF-9909-treated coloring inorganic pigments, W: white, R: red, B: black (number average primary particle diameter: 210 to 400 nm), Shin-Etsu Chemical Co., Ltd. (Note 3) Triethoxysilylethyl polydimethylsiloxyethylhexyl dimethicone, Shin-Etsu Chemical Co., Ltd. | | |

### (Production method)

A: Components 10 and 11 were heated and mixed, and components 12 to 18 were dispersed with a roll mill to give a dispersion of the present invention.
B: Components 1 to 9 were mixed uniformly at 90°C.
C: The dispersion obtained in A and component 19 were added to the mixture obtained in B. The resultant mixture was mixed uniformly at 80°C, poured into a mold, and then slowly cooled to give a lip cosmetic.

The lip cosmetic obtained as described above showed high stability, a non-sticky pleasant feeling on use, and excellent water resistance. The dispersion of the present invention prepared in the production step A had excellent stability and was easy to handle and admit. The pigment paste had high affinity with the lips and was highly resistant to secondary cosmetic transfer.

### [Example 37] Aqueous gel

| Formulation | | % |
|---|---|---|
| 1. | Dispersion of Example 14 | 5.0 |
| 2. | Ethanol | 3.5 |
| 3. | BG | 4.0 |
| 4. | Glycerin | 2.0 |
| 5. | (Ammonium acryloyldimethyltaurate/VP) copolymer | 0.2 |
| 6. | Xanthan gum | 0.2 |
| 7. | KSP-100W (Note 1) | 1.0 |
| 8. | Phenoxyethanol | 0.3 |
| 9. | Water | Balance |
| Total | | 100.0 |

| | | |
|---|---|---|
| (Note 1) Hydrophilized powder of (vinyl dimethicone/methicone silsesquioxane) crosspolymer, Shin-Etsu Chemical Co., Ltd. | | |

### (Production method)

A: Components 1 and 2 were mixed uniformly.
B: Components 3 to 9 were mixed uniformly.
C: The mixture obtained in A was added to the mixture obtained in B. The resultant mixture was mixed uniformly.
D: The mixture obtained in C was defoamed and then added into a container. An aqueous gel was thus obtained.

The aqueous gel obtained as described above was an aqueous gel (cosmetic composition) having high stability, excellent water resistance, and good feeling on use (non-stickiness).

### [Example 38] Oil-in-water cream

| Formulation | | % |
|---|---|---|
| 1. | KSG-15 (Note 1) | 8.0 |
| 2. | KSG-16 (Note 2) | 19.0 |
| 3. | KSG-45 (Note 3) | 5.0 |
| 4. | Cyclopentasiloxane | 10.0 |
| 5. | BG | 3.0 |
| 6. | KF-6100 (Note 4) | 0.6 |
| 7. | KF-6104 (Note 5) | 0.3 |
| 8. | Aqueous (ammonium acryloyldimethyltaurate/VP) copolymer solution (Note 6) | 13.0 |
| 9. | Aqueous (acrylamide/sodium acryloyldimethyltaurate)copolymer/ isohexadecane/polysorbate 80 solution (Note 7) | 0.6 |
| 10. | 1% Aqueous sodium chloride solution | 8.0 |
| 11. | Water | Balance |
| 12. | Dispersion of Example 2 | 6.0 |
| 13. | KM-116 | 10.0 |
| Total | | 100.0 |

| | | |
|---|---|---|
| (Note 1) Mixture of 90-96% cyclopentasiloxane + 4-10% (dimethicone/vinyl dimethicone) crosspolymer, Shin-Etsu Chemical Co., Ltd. (Note 2) Mixture of 70-80% dimethicone + 20-30% (dimethicone/vinyl dimethicone) crosspolymer, Shin-Etsu Chemical Co., Ltd. (Note 3) Mixture of 60-70% cocoalkyl (caprylate/caprate) + 30-40% (vinyl dimethicone/ lauryl dimethicone) crosspolymer, Shin-Etsu Chemical Co., Ltd. (Note 4) Polyglyceryl-3 disiloxane dimethicone, Shin-Etsu Chemical Co., Ltd. (Note 5) Polyglyceryl-3 polydimethylsiloxyethyl dimethicone, Shin-Etsu Chemical Co., Ltd. (Note 6) Aristoflex AVC, Clariant AG (Note 7) SIMULGEL 600, Seppic | | |

### (Production method)

A: Components 1 to 4 were mixed uniformly.
B: Components 5 to 11 were mixed uniformly.
C: The mixture obtained in A was added to the mixture obtained in B and emulsified, and components 12 and 13 were added. The resultant mixture was uniformly dispersed.

The oil-in-water cream obtained as described above was a cosmetic composition having high stability, excellent water resistance, and good feeling on use (non-stickiness).

### [Example 39] Water-in-oil cream

| Formulation | % |
|---|---|
| 1. KSG-710 (Note 1) | 6 |
| 2. KSG-19 (Note 2) | 2 |
| 3. Dimethicone (6 cs) | 13.5 |
| 4. KF-4418 (Note 3) | 5 |
| 5. KF-6104 (Note 4) | 1 |
| 6. KF-6105 (Note 5) | 1 |
| 7. Dispersion of Example 3 | 3 |
| 8. Sodium citrate | 0.2 |
| 9. DPG | 8 |
| 10. Diglycerin | 3 |
| 11. Water | Balance |
| Total | 100.0 |

| | |
|---|---|
| (Note 1) Mixture of dimethicone (6 cs) + 20-30% (dimethicone/polyglycerin-3) crosspolymer, Shin-Etsu Chemical Co., Ltd. (Note 2) Mixture of dimethicone (6 cs) + 10-20% (dimethicone/vinyl dimethicone) crosspolymer, Shin-Etsu Chemical Co., Ltd. (Note 3) Caprylyl methicone, Shin-Etsu Chemical Co., Ltd. (Note 4) Polyglyceryl-3 polydimethylsiloxyethyl dimethicone, Shin-Etsu Chemical Co., Ltd. (Note 5) Lauryl polyglyceryl-3 polydimethylsiloxyethyl dimethicone, Shin-Etsu Chemical Co., Ltd. | |

### (Production method)

A: Components 1 to 6 were mixed uniformly.
B: Components 7 to 11 were mixed uniformly.
C: While performing stirring, the mixture obtained in B was gradually added to the mixture obtained in A and emulsified.

The water-in-oil cream obtained as described above was a cosmetic composition having high stability, excellent water resistance, and good feeling on use (non-stickiness).

### [Example 40] Oil-in-water sunscreen milky lotion

| Formulation | % |
|---|---|
| 1. KTP-09W (Note 1) | 7.5 |
| 2. Ethanol | 0.5 |
| 3. BG | 1.0 |
| 4. KS-66 (Note 2) | 0.001 |
| 5. Water | 4.999 |
| 6. Polyglyceryl-3 disiloxane dimethicone (HLB 10) | 1.2 |
| 7. Ethylhexylglycerin | 0.1 |
| 8. Ethanol | 9.5 |
| 9. BG | 5.0 |
| 10. Sodium acrylate/sodium acryloyldimethyltaurate copolymer composition (Note 3) | 2.5 |
| 11. KSP-100W (Note 4) | 2.0 |
| 12. Water | Balance |
| 13. KF-7312J (Note 5) | 1.0 |
| 14. KF-56A (Note 6) | 3.0 |
| 15. Cetanol | 2.0 |
| 16. Ethylhexyl methoxycinnamate | 5.0 |
| 17. Diethylamino hydroxybenzoyl hexyl benzoate | 1.0 |
| 18. Polyoxyethylene (60) hydrogenated castor oil | 1.0 |
| 19. KF-6011 (Note 7) | 0.5 |
| 20. Tocopherol | 0.05 |
| Total | 100.0 |

| | |
|---|---|
| (Note 1) KF-9909-treated titanium dioxide, Shin-Etsu Chemical Co., Ltd. (Note 2) Simethicone, Shin-Etsu Chemical Co., Ltd. (Note 3) SIMULGEL EG, SEPPIC (Note 4) Hydrophilized powder of (vinyl dimethicone/methicone silsesquioxane) crosspolymer, Shin-Etsu Chemical Co., Ltd. (Note 5) 50% Solution of trimethylsiloxysilicic acid in cyclopentasiloxane, Shin-Etsu Chemical Co., Ltd. (Note 6) Diphenylsiloxyphenyl trimethicone, Shin-Etsu Chemical Co., Ltd. (Note 7) PEG-11 methyl ether dimethicone, Shin-Etsu Chemical Co., Ltd. | |

### (Production method)

A: Components 1 to 7 were dispersed uniformly using a homomixer.
B: Components 8 to 12 were heated to 85°C, and the mixture obtained in A was added. The resultant mixture was mixed uniformly.
C: Components 13 to 20 were heated to 85°C and mixed uniformly.
D: The mixture obtained in C was added to the mixture obtained in B and emulsified at 85°C. While performing stirring, the emulsion was cooled slowly to give an oil-in-water sunscreen milky lotion.

The oil-in-water sunscreen milky lotion obtained as described above showed high stability, a non-sticky pleasant feeling on use, and excellent water resistance. The dispersion of the present invention prepared in the production method A was excellent in dispersibility, particularly in dispersibility in an aqueous medium, water resistance, and feeling on use, and was easy to handle and admit.

### [Example 41] W/O shaking sunscreen

| Formulation | % |
|---|---|
| 1. KSG-15 (Note 1) | 2 |
| 2. KSG-16 (Note 2) | 4 |
| 3. KF-6104 (Note 3) | 0.5 |
| 4. Cyclopentasiloxane | Balance |
| 5. Dimethylpolysiloxane (6 cs) | 3 |
| 6. Isotridecyl isononanoate | 4.8 |
| 7. KSP-105 (Note 4) | 1 |
| 8. BG | 2 |
| 9. Phenoxyethanol | Proper |
| 10. Sodium citrate | Proper |
| 11. Sodium chloride | Proper |
| 12. Water | 13 |
| 13. SPD-T7 (Note 5) | 10 |
| 14. SPD-Z5 (Note 6) | 10 |
| 15. Dispersion of Example 2 | 3 |
| Total | 100.0 |

| | |
|---|---|
| (Note 1) Mixture of 90-96% cyclopentasiloxane + 4-10% (dimethicone/vinyl dimethicone) crosspolymer, Shin-Etsu Chemical Co., Ltd. (Note 2) Mixture of 70-80% dimethicone + 20-30% (dimethicone/vinyl dimethicone) crosspolymer, Shin-Etsu Chemical Co., Ltd. (Note 3) Polyglyceryl-3 polydimethylsiloxyethyl dimethicone, Shin-Etsu Chemical Co., Ltd. (Note 4) (Vinyl dimethicone/methicone silsesquioxane) crosspolymer, Shin-Etsu Chemical Co., Ltd. (Note 5) 45% Dispersion of fine particle of titanium dioxide in cyclopentasiloxane, Shin-Etsu Chemical Co., Ltd. (Note 6) 60% Dispersion of fine particle of zinc oxide in cyclopentasiloxane, Shin-Etsu Chemical Co., Ltd. | |

### (Production method)

A: Components 1 to 7 were mixed uniformly.
B: Components 8 to 12 were mixed uniformly.
C: The mixture obtained in B was added to the mixture obtained in A and emulsified.
D: Components 13, 14, and 15 were added to the emulsion obtained in C to give a W/O shaking sunscreen.

The W/O shaking sunscreen thus obtained showed high stability, a non-sticky pleasant feeling on use, and excellent water resistance.

### [Example 42] Oil-in-water liquid foundation

| Formulation | % |
|---|---|
| 1. Stearic acid | 1.0 |
| 2. Behenyl alcohol | 0.4 |
| 3. Glyceryl stearate | 0.3 |
| 4. Mineral oil | 10.0 |
| 5. Glyceryl trioctanoate | 5.0 |
| 6. Sorbitan sesquioleate | 0.5 |
| 7. Sorbitan monooleate | 1.0 |
| 8. KF-7312T (Note 1) | 1.0 |
| 9. KSP-105 (Note 2) | 1.0 |
| 10. SPD-Z7L (Note 3) | 5.0 |
| 11. Acrylates copolymer | 2.2 |
| 12. Triethanolamine | 1.0 |
| 13. KF-6013 (Note 4) | 0.2 |
| 14. Alkyl-POE palmityl ether phosphate | 0.1 |
| 15. POE hydrogenated castor oil | 0.5 |
| 16. Triethoxycaprylylsilane-treated titanium dioxide (Note 5) | 8.5 |
| 17. Triethoxycaprylylsilane-treated red iron oxide (Note 5) | 0.4 |
| 18. Triethoxycaprylylsilane-treated yellow iron oxide (Note 5) | 1.0 |
| 19. Triethoxycaprylylsilane-treated black iron oxide (Note 5) | 0.1 |
| 20. DPG | 6.0 |
| 21. Polyglyceryl-3 disiloxane dimethicone (HLB 10) | 1.0 |
| 22. Water | 3.0 |
| 23. BG | 7.0 |
| 24. Preservative | Proper |
| 25. Fragrance | Proper |
| 26. Water | Balance |
| Total | 100.0 |

| | |
|---|---|
| (Note 1) 60% Solution of trimethylsiloxysilicic acid in methyl trimethicone, Shin-Etsu Chemical Co., Ltd. (Note 2) (Vinyl dimethicone/methicone silsesquioxane) crosspolymer, Shin-Etsu Chemical Co., Ltd. (Note 3) 60% Dispersion of fine particle of zinc oxide in dimethicone, Shin-Etsu Chemical Co., Ltd. (Note 4) PEG-9 dimethicone, Shin-Etsu Chemical Co., Ltd. (Note 5) (Number average primary particle diameter: 210 to 600 nm) | |

### (Production method)

A: Components 13 to 15 were mixed with a portion of component 23. The resultant mixture was heated.
B: Components 1 to 10 were mixed and heated.
C: Components 11 and 12, the remainder of component 23, and components 24 and 26 were mixed and heated.
D: Components 14 to 21 were dispersed using a disperser.
E: While performing stirring, the mixture obtained in B was added to the mixture obtained in C and emulsified. The mixture obtained in A was added, and further the mixture obtained in D and component 25 were added. An oil-in-water liquid foundation was thus obtained.

The oil-in-water liquid foundation obtained as described above showed high stability, a non-sticky pleasant feeling on use, and excellent water resistance. The dispersion of the present invention obtained in the step D had excellent stability and was easy to handle and admit.

### [Example 43] Oil-in-water mascara

| Formulation | % |
|---|---|
| 1. Stearic acid | 1.0 |
| 2. Cetyl alcohol | 0.5 |
| 3. Glyceryl stearate | 0.5 |
| 4. Beeswax | 7.0 |
| 5. Carnauba wax | 2.0 |
| 6. Triethylhexanoin | 3.0 |
| 7. Sorbitan sesquioleate | 0.7 |
| 8. Polysorbate 80 | 1.5 |
| 9. KF-6011P (Note 1) | 0.5 |
| 10. BG | 5.0 |
| 11. Acrylic acid/alkyl copolymer (solid content: 30%) | 2.5 |
| 12. Polyvinyl acetate emulsion (solid content: 50%) | 25.0 |
| 13. Triethanolamine | 0.7 |
| 14. Dispersion of Example 8 | 12.5 |
| 15. Water | Balance |
| Total | 100.0 |

| | |
|---|---|
| (Note 1) PEG-11 methyl ether dimethicone, Shin-Etsu Chemical Co., Ltd. | |

### (Production method)

A: Components 1 to 9 were heated and stirred.
B: Components 10 to 13 and 15 were mixed and heated.
C: The mixture obtained in B was added to the mixture obtained in A and emulsified.
D: Component 14 was added to the emulsion obtained in C. The resultant mixture was mixed to give a dispersion.

The oil-in-water mascara obtained as described above showed high stability, a non-sticky pleasant feeling on use, and excellent water resistance.

### [Example 44] Oil-in-water lip cosmetic

| Formulation | % |
|---|---|
| 1. Hydrogenated lecithin | 0.3 |
| 2. Stearic acid | 1.5 |
| 3. Beeswax | 14.0 |
| 4. Triethanolamine | 1.2 |
| 5. Polysorbate 80 | 0.5 |
| 6. Sorbitan sesquioleate | 0.2 |
| 7. BG | 5.2 |
| 8. (Acrylates/VA) copolymer emulsion (43%) | 12.0 |
| 9. Water | Balance |
| 10. Dispersion of Example 6 | 1.0 |
| 11. Dispersion of Example 3 | 1.0 |
| Total | 100.0 |

### (Production method)

A: Components 1 to 3 were heated at 100°C to give a solution.
B: Component 4 and a portion of component 9 were heated and mixed at 90°C.
C: The mixture obtained in B was added to the mixture obtained in A and emulsified.
D: Component 5 to the remainder of component 9 were mixed and added to the mixture obtained in C. The resultant mixture was mixed.
E: Components 10 and 11 were mixed and added to the mixture obtained in D. The resultant mixture was mixed.

The oil-in-water lip cosmetic obtained as described above showed high stability, a non-sticky pleasant feeling on use, and excellent water resistance.

### [Example 45] Oil-in-water eye color

| Formulation | % |
|---|---|
| 1. Sorbitan sesquioleate | 0.5 |
| 2. Polyoxyethylene sorbitan monooleate | 1 |
| 3. Dimethicone 2 cs | 5 |
| 4. Ca/Al borosilicate | 3 |
| 5. Organic pigment | Proper |
| 6. Glyceryl stearate | 0.5 |
| 7. Dextrin palmitate | 1 |
| 8. Candelilla wax | 5 |
| 9. Vaseline | 2 |
| 10. KF-7312L (Note 1) | 1.5 |
| 11. KMP-591 (Note 2) | 2 |
| 12. (Ammonium acryloyldimethyltaurate/VP) copolymer | 1 |
| 13. BG | 1 |
| 14. Xanthan gum | 5 |
| 15. Silica | 3 |
| 16. Triethanolamine | 1.2 |
| 17. EDTA | 0.1 |
| 18. Preservative | Proper |
| 19. Dispersions of Examples 6. 7. and 8 | 0.25 |
| Total | 100.0 |

| | |
|---|---|
| (Note 1) 50% Solution of trimethylsiloxysilicic acid in dimethicone 2 cs, Shin-Etsu Chemical Co., Ltd. (Note 2) Polymethylsilsesquioxane, Shin-Etsu Chemical Co., Ltd. | |

### (Production method)

A: Components 6 to 11 were heated at 100°C to give a solution.
B: Components 1 to 5 were added to the mixture obtained in A. The resultant mixture was mixed.
C: Components 12 to 18 were mixed. The resultant mixture was added to the mixture obtained in B and emulsified.
D: Component 19 was added to the emulsion obtained in C. The resultant mixture was mixed.

The oil-in-water eye color obtained as described above showed high stability, a non-sticky pleasant feeling on use, and excellent water resistance.

### [Example 46] Oil-in-water base cream

| Formulation | % |
|---|---|
| 1. Water | Balance |
| 2. Glycerin | 3.0 |
| 3. Microcrystalline wax | 3.0 |
| 4. Xanthan gum | 0.2 |
| 5. Pentylene glycol | 2.0 |
| 6. BG | 5.0 |
| 7. KTP-09W | 5.0 |
| 8. BG | 1.0 |
| 9. Polyglyceryl-3 polydimethylsiloxyethyl dimethicone (HLB 12) (Note 1) | 0.1 |
| 10. Polyglyceryl-3 disiloxane dimethicone (HLB 10) | 0.7 |
| 11. Water | 3.3 |
| 12. Sucrose cocoate | 0.2 |
| 13. Sorbitan stearate | 3.0 |
| 14. PEG-60 glyceryl isostearate | 0.5 |
| 15. Behenyl alcohol | 0.5 |
| 16. Ethylhexyl palmitate | 3.0 |
| 17. KSP-101 (Note 2) | 3.0 |
| 18. Polysorbate 60 | 0.3 |
| 19. (Hydroxyethyl acrylate/sodium acryloyldimethyltaurate) copolymer (Note 4) | 0.6 |
| Total | 100.0 |

| | |
|---|---|
| (Note 1) Soluble in water and BG (Note 2) (Vinyl dimethicone/methicone silsesquioxane) crosspolymer, Shin-Etsu Chemical Co., Ltd. (Note 3) SIMULGEL EG, SEPPIC | |

### (Production method)

A: Components 7 to 11 were dispersed uniformly with a roll.
B: Components 1 to 6 were mixed uniformly, and the dispersion obtained in A was added. The resultant mixture was mixed uniformly.
C: Components 12 to 16 were heated and dissolved, and component 17 was added. The resultant mixture was mixed uniformly.
D: The hot mixture obtained in C was added to the hot mixture obtained in B. The resultant mixture was emulsified uniformly.
E: The emulsion obtained in D was cooled to room temperature, and subsequently components 18 and 19 were added. The resultant mixture was mixed uniformly, defoamed, and then added into a container.

The oil-in-water base cream obtained as described above showed high stability, a non-sticky pleasant feeling on use, and excellent water resistance. The dispersion of the present invention obtained in the step A was excellent in dispersibility, particularly in dispersibility in an aqueous medium, water resistance, and feeling on use, and was easy to handle and admit.

### [Example 47] W/O concealer

| Formulation | % |
|---|---|
| 1. KSG-790 (Note 1) | 6 |
| 2. KSG-44 (Note 2) | 25 |
| 3. KSG-15 (Note 3) | 15 |
| 4. KF-6180 (Note 4) | 1 |
| 5. KF-4422 (Note 5) | 13 |
| 6. Hexyl laurate | 5 |
| 7. Magnesium sulfate | 0.4 |
| 8. Sodium citrate | 0.1 |
| 9. Glycerin | 2 |
| 10. Pentylene glycol | 2 |
| 11. Water | 17.5 |
| 12. Dispersion of Example 9 | 10 |
| 13. Silicone-treated talc | 2.4 |
| 14. BG | 0.45 |
| 15. Polyglyceryl-3 disiloxane dimethicone (HLB 10) | 0.15 |
| Total | 100.0 |

| | |
|---|---|
| (Note 1) Mixture of 65-75% caprylyl methicone + 25-35% (dimethicone/polyglycerin-3) crosspolymer, Shin-Etsu Chemical Co., Ltd. (Note 2) Mixture of 65-75% squalane + 25-35% (vinyl dimethicone/lauryl dimethicone) crosspolymer, Shin-Etsu Chemical Co., Ltd. (Note 3) Mixture of 90-96% cyclopentasiloxane + 4-10% (dimethicone/vinyl dimethicone) crosspolymer, Shin-Etsu Chemical Co., Ltd. (Note 4) Isostearyl polyglyceryl-3 dimethicone, Shin-Etsu Chemical Co., Ltd. (Note 5) Ethyl methicone, Shin-Etsu Chemical Co., Ltd. | |

### (Production method)

A: Components 13 to 15 were uniformly dispersed using a disperser.
B: Components 1 to 6 were mixed uniformly.
C: Components 8 to 12 were mixed uniformly. The resultant mixture was added gradually to the mixture obtained in B and emulsified.
D: Component 12 and the mixture obtained in A were mixed uniformly into the mixture obtained in C.

The W/O concealer obtained as described above showed high stability, a non-sticky pleasant feeling on use, and excellent water resistance.

### [Example 48] Aqueous eye liner

| Formulation | % |
|---|---|
| 1. Alkyl acrylate copolymer emulsion | 15 |
| 2. Ethanol | 2 |
| 3. Methylparaben | 0.1 |
| 4. Water | 49.8 |
| 5. BG | 15 |
| 6. Xanthan gum | 0.1 |
| 7. Dispersion of Example 17 | 18 |
| Total | 100.0 |

### (Production method)

A: Components 1 to 6 were mixed uniformly.
B: Component 7 was mixed uniformly into the mixture obtained in A.

The aqueous eye liner obtained as described above showed high stability, a non-sticky pleasant feeling on use, and excellent water resistance.

### [Example 49] W/O cream foundation

| | Components | % |
|---|---|---|
| 1. | KSG-240 (Note 1) | 3 |
| 2. | KSG-15 (Note 2) | 7 |
| 3. | KF-6038 (Note 3) | 3 |
| 4. | Cyclopentasiloxane | Balance |
| 5. | Ethylhexyl methoxycinnamate | 4 |
| 6. | Diethylamino hydroxybenzoyl hexyl benzoate | 1 |
| 7. | Organically modified clay mineral | 1.2 |
| 8. | KP-549 (Note 4) | 3 |
| 9. | KF-56A (Note 5) | 10 |
| 10. | Isotridecyl isononanoate | 7 |
| 11. | Dispersion of Example 9 | 12.5 |
| 12. | DPG | 5 |
| 13. | Phenoxyethanol | 0.2 |
| 14. | Sodium citrate | Proper |
| 15. | Magnesium sulfate | Proper |
| 16. | Water | 30 |
| | Total | 100.0 |

| | | |
|---|---|---|
| (Note 1) Mixture of 75-85% cyclopentasiloxane + 15-25% (dimethicone/(PEG-10/15)) crosspolymer, Shin-Etsu Chemical Co., Ltd. (Note 2) Mixture of 90-96% cyclopentasiloxane + 4-10% (dimethicone/vinyl dimethicone) crosspolymer, Shin-Etsu Chemical Co., Ltd. (Note 3) Lauryl PEG-9 polydimethylsiloxyethyl dimethicone, Shin-Etsu Chemical Co., Ltd. (Note 4) 40% Solution of (acrylates/dimethicone) copolymer in methyl trimethicone, Shin-Etsu Chemical Co., Ltd. (Note 5) Diphenylsiloxyphenyl trimethicone, Shin-Etsu Chemical Co., Ltd. | | |

### (Production method)

A: Components 1 to 10 were mixed uniformly.
B: Components 11 to 16 were mixed uniformly.
C: The mixture obtained in B was added to the mixture obtained in C and emulsified.

The W/O cream foundation thus obtained showed high stability, a non-sticky pleasant feeling on use, and excellent water resistance.

### [Example 50] W/O sunscreen milky lotion

| | Formulation | % |
|---|---|---|
| 1. | KSG-270 (Note 1) | 3.0 |
| 2. | KSG-18A (Note 2) | 3.0 |
| 3. | KF-6048 (Note 3) | 2.0 |
| 4. | KF-56A (Note 4) | 5.0 |
| 5. | KF-4422 (Note 5) | Balance |
| 6. | Ethylhexyl methoxycinnamate | 5.0 |
| 7. | Ethylhexyl salicylate | 2.0 |
| 8. | Octocrylene | 1.0 |
| 9. | Diethylamino hydroxybenzoyl hexyl benzoate | 2.0 |
| 10. | KSP-105 (Note 6) | 2.0 |
| 11. | SPD-T7 (Note 7) | 10.0 |
| 12. | SPD-Z5 (Note 8) | 10.0 |
| 13. | BG | 3.0 |
| 14. | Ethanol | 5.0 |
| 15. | Sodium citrate | 0.2 |
| 16. | Sodium chloride | 0.5 |
| 17. | KSP-100W (Note 9) | 3.0 |
| 18. | Water | 17.0 |
| 19. | Dispersion of Example 2 | 1.0 |
| | Total | 100.0 |

| | | |
|---|---|---|
| (Note 1) Mixture of diphenylsiloxyphenyl trimethicone + 15-25% (dimethicone/(PEG-10/15)) crosspolymer, Shin-Etsu Chemical Co., Ltd. (Note 2) Mixture of diphenylsiloxyphenyl trimethicone + 10-20% (dimethicone/phenylvinyl dimethicone) crosspolymer, Shin-Etsu Chemical Co., Ltd. (Note 3) Cetyl PEG/PPG-10/1 dimethicone, Shin-Etsu Chemical Co., Ltd. (Note 4) Diphenylsiloxyphenyl trimethicone, Shin-Etsu Chemical Co., Ltd. (Note 5) Ethyl trimethicone, Shin-Etsu Chemical Co., Ltd. (Note 6) (Vinyl dimethicone/methicone silsesquioxane) crosspolymer, Shin-Etsu Chemical Co., Ltd. (Note 7) 45% Dispersion of fine particle of titanium dioxide in cyclopentasiloxane, Shin-Etsu Chemical Co., Ltd. (Note 8) 60% Dispersion of fine particle of zinc oxide in cyclopentasiloxane, Shin-Etsu Chemical Co., Ltd. (Note 9) Hydrophilized powder of (vinyl dimethicone/methicone silsesquioxane) crosspolymer, Shin-Etsu Chemical Co., Ltd. | | |

### (Production method)

A: Components 1 to 10 were mixed uniformly.
B: Components 13 to 18 were mixed uniformly, and component 19 was admixed.
C: The mixture obtained in B was added to the mixture obtained in A and emulsified.
D: Components 11 and 12 were added to the mixture obtained in C. The resultant mixture was mixed uniformly.

The W/O sunscreen milky lotion thus obtained showed high stability, a non-sticky pleasant feeling on use, and excellent water resistance.

### [Example 51] Spray foundation

| | Formulation | % |
|---|---|---|
| 1. | Glyceryl stearate | 1 |
| 2. | Stearic acid | 3 |
| 3. | Behenyl alcohol | 0.5 |
| 4. | Cetanol | 0.5 |
| 5. | Squalane | 3 |
| 6. | Talc | 1 |
| 7. | Lecithin | 0.3 |
| 8. | BG | 13 |
| 9. | Triethanolamine | 1.5 |
| 10. | Ethanol | 5 |
| 11. | Water | Balance |
| 12. | Xanthan gum (2% aqueous solution) | 12 |
| 13. | Dispersion of Example 9 | 3 |
| | Total | 100.0 |

### (Production method)

A: Components 1 to 6 were mixed uniformly at 80°C.
B: Components 7 to 11 were mixed uniformly at 80°C.
C: The mixture obtained in B was added to the mixture obtained in A and emulsified, and components 12 and 13 were admixed at 25°C.
D: The mixture obtained in C was added into an aerosol container together with a propellant.

The spray foundation thus obtained showed high stability, a non-sticky pleasant feeling on use, and excellent water resistance.

### [Example 52] Aqueous sunscreen mist

| | Formulation | % |
|---|---|---|
| 1. | Dispersion of Example 2 | 1.0 |
| 2. | Hydrous silica-coated silicone-treated fine particle of titanium dioxide | 7.0 |
| 3. | Polyglyceryl-3 disiloxane dimethicone (HLB 10) | 1.4 |
| 4. | BG | 1.4 |
| 5. | Water | Balance |
| 6. | Sodium citrate | 0.1 |
| 7. | Citric acid | 0.02 |
| 8. | Ethanol | 5.0 |
| 9. | BG | 4.0 |
| 10. | Glycerin | 2.0 |
| 11. | Fragrance | 0.2 |
| 12. | Ethylhexylglycerin | 0.1 |
| | Total | 100.0 |

### (Production method)

A: Components 2 to 4 and a portion of component 5 were uniformly dispersed with a bead mill.
B: The mixture obtained in A, components 1, 6, and 7, and the remainder of component 5 were mixed uniformly.
C: Components 8 to 12 were mixed uniformly.
D: The mixture obtained in C was added to the mixture obtained in B. The resultant mixture was mixed uniformly and added into a container.

The aqueous sunscreen mist obtained as described above showed high stability, a non-sticky pleasant feeling on use, and excellent water resistance.

### [Example 53] Aqueous lip color

| | Formulation | % |
|---|---|---|
| 1. | Carbomer | 0.5 |
| 2. | BG | 6 |
| 3. | Glycerin | 8 |
| 4. | PEG-150 | 2 |
| 5. | PEG-60 hydrogenated castor oil | 0.5 |
| 6. | Dimethicone 6 cs | 0.3 |
| 7. | Cyclopentasiloxane | 1.4 |
| 8. | Ceteth-30 | 0.1 |
| 9. | Acrylates copolymer (30%) | 10 |
| 10. | Preservative | Proper |
| 11. | Water | Balance |
| 12. | Potassium hydroxide | 0.2 |
| 13. | KF-9909 (Note 1)-treated talc | 3 |
| 14. | KF-9909 (Note 1)-treated mica | 7 |
| 15. | KTP-09W, R, Y, B (Note 2) | 10 |
| 16. | Polyglyceryl-3 disiloxane dimethicone (HLB 10) | 1.3 |
| 17. | BG | 4 |
| | Total | 100.0 |

| | | |
|---|---|---|
| (Note 1) Triethoxysilylethyl polydimethylsiloxyethylhexyl dimethicone, Shin-Etsu Chemical Co., Ltd. (Note 2) KF-9909-treated pigments, Shin-Etsu Chemical Co., Ltd. | | |

### (Production method)

A: Components 13 to 17 were uniformly dispersed using a roll.
B: Components 1 to 12 were mixed uniformly.
C: The mixture obtained in A was mixed uniformly with the mixture obtained in B.

The aqueous lip color obtained as described above showed high stability, a non-sticky pleasant feeling on use, and excellent water resistance. The dispersion of the present invention obtained in the step A was excellent in dispersibility, particularly in dispersibility in an aqueous medium, water resistance, and feeling on use, and was easy to handle and admit.

### [Example 54] O/W lip tint

| | Formulation | % |
|---|---|---|
| 1. | KSG-45 (Note 1) | 8.5 |
| 2. | Squalane | 10 |
| 3. | Red #218 | 0.05 |
| 4. | Red #227 | Proper |
| 5. | Yellow #5 | Proper |
| 6. | Sodium acrylate-grafted starch | 0.2 |
| 7. | BG | 5 |
| 8. | Preservative | Proper |
| 9. | Water | Balance |
| 10. | Dispersion of Example 15 | 0.5 |
| 11. | Dispersion of Example 18 | 0.2 |
| 12. | Acrylates copolymer emulsion | 5 |
| | Total | 100.0 |

| | | |
|---|---|---|
| (Note 1) Mixture of 60-70% cocoalkyl (caprylate/caprate) + 30-40% (vinyl dimethicone/ lauryl dimethicone) crosspolymer, Shin-Etsu Chemical Co., Ltd. | | |

### (Production method)

A: Components 1 to 5 were mixed uniformly.
B: Components 6 to 9 were mixed uniformly.
C: The mixture obtained in A was mixed uniformly with the mixture obtained in B, and components 10 to 12 were admixed uniformly.

The O/W lip tint obtained as described above showed high stability, a non-sticky pleasant feeling on use, and excellent water resistance.

### [Example 55] Oil-in-water liquid foundation

| | Formulation | % |
|---|---|---|
| 1. | Polyglyceryl-10 isostearate | 3.0 |
| 2. | NBN-30-ID (Note 1) | 2.0 |
| 3. | Behenyl alcohol | 2.0 |
| 4. | Dicaprylyl ether | 2.5 |
| 5. | Octyldodecyl myristate | 5.0 |
| 6. | Dimethicone (20 cs) | 0.5 |
| 7. | (C9-12) alkane | 5.0 |
| 8. | KF-6115 (Note 2) | 0.2 |
| 9. | KTP-09W (Note 3) | 2.1 |
| 10. | KTP-09Y (Note 3) | 0.26 |
| 11. | KTP-09R (Note 3) | 0.11 |
| 12. | KTP-09B (Note 3) | 0.03 |
| 13. | Ethylhexylglycerin | 0.1 |
| 14. | Glycerin | 3.5 |
| 15. | BG | 3.0 |
| 16. | Water | Balance |
| 17. | Xanthan gum | 0.2 |
| 18. | (Ammonium acryloyldimethyltaurate/VP) copolymer | 0.2 |
| 19. | Dispersion of Example 9 | 6.0 |
| | Total | 100.0 |

| | | |
|---|---|---|
| (Note 1) 30% Solution of (norbornene/tris(trimethylsiloxy)silylnorbornene) copolymer in isododecane, Shin-Etsu Chemical Co., Ltd. (Note 2) Lauryl polyglyceryl-3 polydimethylsiloxyethyl dimethicone, Shin-Etsu Chemical Co., Ltd. (Note 3) KF-9909-treated coloring inorganic pigments, W: white, Y: yellow, R: red, B: black (number average primary particle diameter: 210 to 400 nm), Shin-Etsu Chemical Co., Ltd. | | |

### (Production method)

A: Components 1 to 6 were heated and mixed uniformly.
B: Components 13 to 18 were mixed and heated.
C: Components 7 to 12 were mixed uniformly and roll-treated.
D: The mixture obtained in C was added to the mixture obtained in A. The resultant mixture was mixed uniformly.
E: While performing stirring, the mixture obtained in D was added to the mixture obtained in B and emulsified. Component 19 was added. The resultant mixture was mixed to give an oil-in-water liquid foundation.

The oil-in-water liquid foundation obtained as described above showed high stability, a non-sticky pleasant feeling on use, and excellent water resistance. The dispersion of Example 9 had excellent stability and was easy to handle and admit.

### [Example 56] Water-in-oil liquid foundation

| | Formulation | % |
|---|---|---|
| 1. | KSG-330 (Note 1) | 3 |
| 2. | KSG-18A (Note 2) | 5 |
| 3. | KF-6038 (Note 3) | 3 |
| 4. | Dimethicone (2 cs) | Balance |
| 5. | Ethylhexyl methoxycinnamate | 4 |
| 6. | Diethylamino hydroxybenzoyl hexyl benzoate | 1 |
| 7. | Organically modified clay mineral | 1.2 |
| 8. | TSPL-30-ID (Note 4) | 2 |
| 9. | KF-56A (Note 5) | 10 |
| 10. | Ethylhexyl palmitate | 7 |
| 11. | KP-578 (Note 6) | 0.15 |
| 12. | KTP-09W (Note 7) | 4.3 |
| 13. | KTP-09Y (Note 7) | 0.5 |
| 14. | KTP-09R (Note 7) | 0.2 |
| 15. | KTP-09B (Note 7) | 0.1 |
| 16. | Dispersion of Example 9 | 4.0 |
| 17. | BG | 5 |
| 18. | Phenoxyethanol | 0.2 |
| 19. | Sodium citrate | Proper |
| 20. | Sodium chloride | Proper |
| 21. | Water | 30 |
| | Total | 100.0 |

| | | |
|---|---|---|
| (Note 1) Mixture of 75-85% triethylhexanoin + 15-25% (PEG-15/lauryl dimethicone) crosspolymer, Shin-Etsu Chemical Co., Ltd. (Note 2) Mixture of 80-90% diphenylsiloxyphenyl trimethicone + 10-20% (dimethicone/ phenylvinyl dimethicone) crosspolymer, Shin-Etsu Chemical Co., Ltd. (Note 3) Lauryl PEG-9 polydimethylsiloxyethyl dimethicone, Shin-Etsu Chemical Co., Ltd. (Note 4) 30% Solution of pullulan tri(trimethylsiloxy)silylpropyl carbamide in isododecane, Shin-Etsu Chemical Co., Ltd. (Note 5) Diphenylsiloxyphenyl trimethicone, Shin-Etsu Chemical Co., Ltd. (Note 6) (Acrylates/ethylhexyl acrylate/dimethicone methacrylate) copolymer, Shin-Etsu Chemical Co., Ltd. (Note 7) KF-9909-treated coloring inorganic pigments, W: white, Y: yellow, R: red, B: black (number average primary particle diameter: 210 to 400 nm), Shin-Etsu Chemical Co., Ltd. | | |

### (Production method)

A: Components 1 to 9 were mixed. The resultant mixture was heated and mixed uniformly.
B: Components 16 to 21 were mixed uniformly.
C: Components 10 to 15 were mixed uniformly and treated with a homomixer.
D: While performing stirring, the mixture obtained in B was added to the mixture obtained in A and emulsified. The mixture obtained in C was added. The resultant mixture was mixed to give a water-in-oil liquid foundation.

The water-in-oil liquid foundation obtained as described above showed high stability, a non-sticky pleasant feeling on use, and excellent water resistance. The dispersion of Example 9 had excellent stability and was easy to handle and admit.

### [Example 57] Oil-in-water liquid foundation

| | Formulation | % |
|---|---|---|
| 1. | Polyglyceryl-10 myristate | 0.5 |
| 2. | Polyglyceryl-10 isostearate | 1.0 |
| 3. | KF-6043 (Note 1) | 1.0 |
| 4. | Diisopropyl sebacate | 2.5 |
| 5. | Ethylhexyl triazone | 0.5 |
| 6. | Tridecane | 5.0 |
| 7. | Dimethicone (6 cs) | 0.5 |
| 8. | KF-6115 (Note 2) | 0.2 |
| 9. | KTP-09W (Note 3) | 2.1 |
| 10. | Ethylhexylglycerin | 0.1 |
| 11. | BG | 2.3 |
| 12. | KF-6100 (Note 4) | 0.2 |
| 13. | KTP-09Y (Note 3) | 0.26 |
| 14. | KTP-09R (Note 3) | 0.11 |
| 15. | KTP-09B (Note 3) | 0.03 |
| 16. | Water | Balance |
| 17. | (Hydroxyethyl acrvlate/sodium acryloyldimethyltaurate) copolymer | 0.2 |
| | Total | 100.0 |

| | | |
|---|---|---|
| (Note 1) PEG-10 dimethicone, Shin-Etsu Chemical Co., Ltd. (Note 2) Lauryl polyglyceryl-3 polydimethylsiloxyethyl dimethicone, Shin-Etsu Chemical Co., Ltd. (Note 3) KF-9909-treated coloring inorganic pigments, W: white, Y: yellow, R: red, B: black (number average primary particle diameter: 210 to 400 nm), Shin-Etsu Chemical Co., Ltd. (Note 4) Polyglyceryl-3 disiloxane dimethicone, Shin-Etsu Chemical Co., Ltd. | | |

### (Production method)

A: Components 1 to 6 were heated and mixed uniformly.
B: Components 10, 16, and 17 were mixed uniformly.
C: Components 7 to 9 were mixed uniformly and roll-treated.
D: Components 11 to 15 were mixed uniformly and roll-treated.
E: The mixture obtained in C was mixed with the mixture obtained in A.
F: While performing stirring, the mixture obtained in E was added to the mixture obtained in B and emulsified. The mixture obtained in D was added. The resultant mixture was mixed to give an oil-in-water liquid foundation.

The oil-in-water liquid foundation obtained as described above showed high stability, a non-sticky pleasant feeling on use, and excellent water resistance.

### [Example 58] Oil-in-water cream foundation

| | Formulation | % |
|---|---|---|
| 1. | Polyglyceryl-10 isostearate | 3.0 |
| 2. | KP-550 (Note 1) | 2.0 |
| 3. | X-21-5595 (Note 2) | 2.0 |
| 4. | Dibutyl adipate | 2.5 |
| 5. | Ethylhexyl triazone | 0.5 |
| 6. | Bis-ethylhexyloxyphenol methoxyphenyl triazine | 0.5 |
| 7. | Dodecane | 5.0 |
| 8. | KF-6106 (Note 3) | 0.2 |
| 9. | KTP-09Y (Note 4) | 0.26 |
| 10. | KTP-09R (Note 4) | 0.11 |
| 11. | KTP-09B (Note 4) | 0.03 |
| 12. | Ethylhexylglycerin | 0.1 |
| 13. | Glycerin | 3.5 |
| 14. | BG | 3.0 |
| 15. | Water | Balance |
| 16. | (PEG-240/decyltetradeceth-20/HDI) copolymer | 0.2 |
| 17. | KTP-09W (Note 4) | 2.1 |
| 18. | BG | 0.45 |
| 19. | Polyglyceryl-3 disiloxane dimethicone (HLB 10) | 0.15 |
| | Total | 100.0 |

| | | |
|---|---|---|
| (Note 1) 40% Solution of (acrylates/dimethicone) copolymer in isododecane, Shin-Etsu Chemical Co., Ltd. (Note 2) 60% Solution of trimethylsiloxysilicic acid in isododecane, Shin-Etsu Chemical Co., Ltd. (Note 3) Polyglyceryl-3 polydimethylsiloxyethyl dimethicone, Shin-Etsu Chemical Co., Ltd. (Note 4) KF-9909-treated coloring inorganic pigments, W: white, Y: yellow, R: red, B: black (number average primary particle diameter: 210 to 400 nm), Shin-Etsu Chemical Co., Ltd. | | |

### (Production method)

A: Components 1 to 6 were heated and mixed uniformly.
B: Components 12 to 16 were heated and mixed uniformly.
C: Components 7 to 11 were mixed uniformly and treated with a high-pressure disperser.
D: Components 17 to 19 were mixed uniformly and roll-treated.
E: The mixture obtained in C was mixed with the mixture obtained in A.
F: The mixture obtained in D was mixed with the mixture obtained in B.
G: While performing stirring, the mixture obtained in E was added to the mixture obtained in F and emulsified to give an oil-in-water cream foundation.

The oil-in-water cream foundation obtained as described above showed high stability, a non-sticky pleasant feeling on use, and excellent water resistance.

### [Example 59] W/O cream foundation

| Formulation | | % |
|---|---|---|
| 1. | KSG-320Z (Note 1) | 6 |
| 2. | KSG-19 (Note 2) | 3 |
| 3. | KSG-43 (Note 3) | 1 |
| 4. | KF-6105 (Note 4) | 1 |
| 5. | Dimethicone (6 cs) | 13 |
| 6. | Squalane | 5 |
| 7. | KF-6115 (Note 5) | 0.2 |
| 8. | KTP-09Y (Note 6) | 0.26 |
| 9. | KTP-09R (Note 6) | 0.11 |
| 10. | KTP-09B (Note 6) | 0.03 |
| 11. | Magnesium sulfate | 0.4 |
| 12. | Sodium citrate | 0.1 |
| 13. | Diglycerin | 2 |
| 14. | Pentylene glycol | 2 |
| 15. | Water | 17.5 |
| 16. | Dispersion of Example 14 | 6 |
| Total | | 100.0 |

| | | |
|---|---|---|
| (Note 1) Mixture of 70-80% isododecane + 20-30% (PEG-15/lauryl polydimethylsiloxyethyl dimethicone) crosspolymer, Shin-Etsu Chemical Co., Ltd. (Note 2) Mixture of 80-90% dimethicone + 10-20% (dimethicone/vinyl dimethicone) crosspolymer, Shin-Etsu Chemical Co., Ltd. (Note 3) Mixture of 65-75% triethylhexanoin + 25-35% (vinyl dimethicone/lauryl dimethicone) crosspolymer, Shin-Etsu Chemical Co., Ltd. (Note 4) Lauryl polyglyceryl-3 polydimethylsiloxyethyl dimethicone, Shin-Etsu Chemical Co., Ltd. (Note 5) Lauryl polyglyceryl-3 polydimethylsiloxyethyl dimethicone, Shin-Etsu Chemical Co., Ltd. (Note 6) KF-9909-treated coloring inorganic pigments, Y: yellow, R: red, B: black (number average primary particle diameter: 210 to 400 nm), Shin-Etsu Chemical Co., Ltd. | | |

### (Production method)

A: Components 1 to 5 were mixed uniformly.
B: Components 11 to 16 were mixed uniformly.
C: Components 6 to 10 were mixed uniformly and treated with a high-pressure disperser.
D: While performing stirring, the mixture obtained in B was added to the mixture obtained in A and emulsified. The mixture obtained in C was added. The resultant mixture was mixed to give a W/O cream foundation.

The W/O cream foundation obtained as described above showed high stability, a non-sticky pleasant feeling on use, and excellent water resistance.

### [Example 60] W/O concealer

| Formulation | | % |
|---|---|---|
| 1. | KSG-840 (Note 1) | 6 |
| 2. | KSG-44 (Note 2) | 25 |
| 3. | KSG-45 (Note 3) | 15 |
| 4. | KF-6180 (Note 4) | 1 |
| 5. | Jojoba seed oil | 13 |
| 6. | Sesame oil | 5 |
| 7. | KF-6115 (Note 5) | 0.2 |
| 8. | KTP-09W (Note 6) | 2.1 |
| 9. | Magnesium sulfate | 0.4 |
| 10. | Sodium citrate | 0.1 |
| 11. | Glycerin | 2 |
| 12. | Pentylene glycol | 2 |
| 13. | Water | 17.5 |
| 14. | KTP-09Y (Note 6) | 0.26 |
| 15. | KTP-09R (Note 6) | 0.11 |
| 16. | KTP-09B (Note 6) | 0.03 |
| 17. | BG | 0.45 |
| 18. | Polyglyceryl-3 disiloxane dimethicone (HLB 10) | 0.15 |
| Total | | 100.0 |

| | | |
|---|---|---|
| (Note 1) Mixture of 65-75% squalane + 25-35% (lauryl dimethicone/polyglycerin-3) crosspolymer, Shin-Etsu Chemical Co., Ltd. (Note 2) Mixture of 65-75% squalane + 25-35% (vinyl dimethicone/lauryl dimethicone) crosspolymer, Shin-Etsu Chemical Co., Ltd. (Note 3) Mixture of 60-70% cocoalkyl (caprylate/caprate) + 30-40% (vinyl dimethicone/ lauryl dimethicone) crosspolymer, Shin-Etsu Chemical Co., Ltd. (Note 4) Isostearyl polyglyceryl-3 dimethicone, Shin-Etsu Chemical Co., Ltd. (Note 5) Lauryl polyglyceryl-3 polydimethylsiloxyethyl dimethicone, Shin-Etsu Chemical Co., Ltd. (Note 6) KF-9909-treated coloring inorganic pigments, W: white, Y: yellow, R: red, B: black (number average primary particle diameter: 210 to 400 nm), Shin-Etsu Chemical Co., Ltd. | | |

### (Production method)

A: Components 1 to 5 were mixed uniformly.
B: Components 9 to 13 were mixed uniformly.
C: Components 6 to 8 were mixed uniformly and then mixed using a disperser.
D: Components 14 to 18 were mixed uniformly and roll-treated.
E: The mixture obtained in D was mixed with the mixture obtained in B.
F: While performing stirring, the mixture obtained in E was added to the mixture obtained in A and emulsified. The mixture obtained in C was added. The resultant mixture was mixed to give a W/O concealer.

The W/O concealer obtained as described above showed high stability, a non-sticky pleasant feeling on use, and excellent water resistance.

### [Example 61] Oil-in-water liquid foundation

| Formulation | | % |
|---|---|---|
| 1. | PEG-60 hydrogenated castor oil | 3.0 |
| 2. | KP-545L (Note 1) | 1.0 |
| 3. | TSPL-30-ID (Note 2) | 1.0 |
| 4. | Isohexadecane | 2.5 |
| 5. | KP-561P (Note 3) | 1.0 |
| 6. | Hydrous silica/hydrogen dimethicone-treated titanium dioxide (Note 4) | 8.1 |
| 7. | KTP-09Y (Note 5) | 0.97 |
| 8. | KTP-09R (Note 5) | 0.41 |
| 9. | KTP-09B (Note 5) | 0.12 |
| 10. | Polyglyceryl-3 disiloxane dimethicone (HLB 10) | 1.5 |
| 11. | BG | 8.0 |
| 12. | Water | Balance |
| 13. | (Hydroxyethyl acrylate/sodium acryloyldimethyltaurate) copolymer | 0.2 |
| Total | | 100.0 |

| | | |
|---|---|---|
| (Note 1) 40% Solution of (acrylates/dimethicone) copolymer in dimethicone, Shin-Etsu Chemical Co., Ltd. (Note 2) 30% Solution of pullulan tri(trimethylsiloxy)silylpropyl carbamide in isododecane, Shin-Etsu Chemical Co., Ltd. (Note 3) (Acrylates/stearyl acrylate/dimethicone methacrylate) copolymer, Shin-Etsu Chemical Co., Ltd. (Note 4) Number average primary particle diameter: 210 to 400 nm (Note 5) KF-9909-treated coloring inorganic pigments, Y: yellow, R: red, B: black (number average primary particle diameter: 210 to 400 nm), Shin-Etsu Chemical Co., Ltd. | | |

### (Production method)

A: Components 1 to 5 were mixed and heated to give a uniform solution.
B: Components 12 and 13 were mixed and heated.
C: Components 6 to 11 were mixed and roll-treated.
D: While performing stirring, the mixture obtained in A was added to the mixture obtained in B and emulsified. The mixture obtained in C was added. The resultant mixture was mixed to give an oil-in-water liquid foundation.

The oil-in-water liquid foundation obtained as described above showed high stability, a non-sticky pleasant feeling on use, and excellent water resistance.

## Claims

1. A dispersion comprising the following components (a) to (d):
(a) 10 to 85% by mass of a hydrophobized inorganic powder having a number average primary particle diameter of more than 200 nm and 700 nm or less as determined by image analysis of a transmission electron photomicrograph;
(b) 1 to 80% by mass of an aqueous component having at least two alcoholic hydroxyl groups;
(c) 1 to 20% by mass of a polyglycerin-modified silicone soluble in the component (b); and
(d) 0 to 2% by mass of a polyglycerin-modified silicone insoluble in the component (b),
the ratio of the content of the component (d) to the content of the component (c), (d)/(c), being 0.1 or less.

2. The dispersion according to claim 1, wherein the component (a) is an inorganic powder hydrophobized with a silicone.

3. The dispersion according to claim 2, wherein the silicone is triethoxysilylethyl polydimethylsiloxyethylhexyl dimethicone.

4. The dispersion according to claim 1, wherein the content of the component (a) is 70 to 85% by mass.

5. The dispersion according to claim 1, wherein the component (a) is a coloring pigment.

6. The dispersion according to claim 1, wherein the component (c) is polyglyceryl-3 disiloxane dimethicone.

7. The dispersion according to claim 1, wherein the content of the component (c) is 3.0 to 8.0% by mass.

8. The dispersion according to claim 1, wherein the ratio of the content of the component (c) to the content of the component (b), (c)/(b), is 0.1 to 0.4.

9. A cosmetic composition comprising the dispersion described in any one of claims 1 to 8.
